(19) **Europäisches Patentamt / European Patent Office / Office européen des brevets**

(11) **EP 4 141 419 B1**

(12) **EUROPEAN PATENT SPECIFICATION**

(45) Date of publication and mention of the grant of the patent:
**12.02.2025 Bulletin 2025/07**

(21) Application number: **21793499.1**

(22) Date of filing: **06.04.2021**

(51) International Patent Classification (IPC):
**G01N 15/1429** *(2024.01)*    **G01N 15/14** *(2024.01)*
**G01N 15/10** *(2024.01)*    **G01N 21/64** *(2006.01)*

(52) Cooperative Patent Classification (CPC):
**G01N 15/1429; G01N 15/1459;** G01N 2015/1006;
G01N 2015/1488; G01N 2021/6421;
G01N 2021/6441

(86) International application number:
**PCT/JP2021/014579**

(87) International publication number:
**WO 2021/215234 (28.10.2021 Gazette 2021/43)**

(54) **INFORMATION PROCESSING DEVICE, INFORMATION PROCESSING METHOD, PROGRAM, AND INFORMATION PROCESSING SYSTEM**

INFORMATIONSVERARBEITUNGSVORRICHTUNG, INFORMATIONSVERARBEITUNGSVERFAHREN, PROGRAMM UND INFORMATIONSVERARBEITUNGSSYSTEM

DISPOSITIF DE TRAITEMENT D'INFORMATIONS, PROCÉDÉ DE TRAITEMENT D'INFORMATIONS, PROGRAMME, ET SYSTÈME DE TRAITEMENT D'INFORMATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.04.2020   JP 2020074671**

(43) Date of publication of application:
**01.03.2023   Bulletin 2023/09**

(73) Proprietor: **Sony Group Corporation**
**Tokyo 108-0075 (JP)**

(72) Inventors:
• **IDE Naoki**
  **Tokyo 108-0075 (JP)**
• **UMETSU Tomoyuki**
  **Tokyo 108-0075 (JP)**

(74) Representative: **D Young & Co LLP**
**3 Noble Street**
**London EC2V 7BQ (GB)**

(56) References cited:
WO-A1-2019/031048    JP-A- 2013 246 140
JP-A- 2014 095 678    JP-A- 2019 070 775
JP-A- H0 844 400    US-A- 5 677 986
US-A1- 2010 012 853    US-A1- 2012 056 103
US-A1- 2016 025 621    US-B1- 8 812 421

• ANYAEGBU CHIDOZIE C ET AL: "Optimisation of multiplex immunofluorescence for a non-spectral fluorescence scanning system", JOURNAL OF IMMUNOLOGICAL METHODS, ELSEVIER SCIENCE PUBLISHERS B.V.,AMSTERDAM, NL, vol. 472, 8 June 2019 (2019-06-08), pages 25 - 34, XP085736316, ISSN: 0022-1759, [retrieved on 20190608], DOI: 10.1016/J.JIM.2019.06.011

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

[Technical Field]

The present technology relates to an information processing system, an information processing method, a program, and an information processing device. More specifically, the present technology relates to an information processing system that performs processing for assigning a phosphor to a biomolecule, an information processing method for the processing, a program for causing an information processing device to execute the processing, and an information processing device included in the information processing system.

[Background Art]

For example, a particle population such as cells is labeled with a fluorescent dye, and intensity and/or a pattern of fluorescence generated from the fluorescent dye excited by irradiating each particle in the particle population with a laser beam is measured in order to measure characteristics of the particles. A typical example of a particle analysis device that performs this measurement is a flow cytometer. Flow cytometers can be roughly classified into a filter type and a spectral type, for example, from the viewpoint of an optical system for fluorescence measurement.

An excitation wavelength with which a signal can be acquired at a high peak is associated with the fluorescent dye. Therefore, in a filter-type flow cytometer of the related art, particles labeled with fluorescent dyes are irradiated with light having an excitation wavelength therefor, and a fluorescent signal is acquired by using an optical filter corresponding to a peak wavelength range of the fluorescent dye.

On the other hand, in a spectral type flow cytometer, for example, fluorescence of respective cells is collectively acquired as a spectrum. Fluorescence separation processing (also referred to as unmixing processing) is performed on the acquired fluorescence spectrum using the spectral reference of each fluorescent dye, and a fluorescence signal is acquired.

As a technology regarding the fluorescence separation processing, for example, PTL 1 below discloses a fluorescence intensity correction method including a procedure of receiving fluorescence generated from a fluorescent dye excited by irradiating microparticles multiplex-labeled with a plurality of fluorescent dyes having overlapping fluorescence wavelength bands with light, by using a larger number of disposed photodetectors having different light reception wavelength bands than the number of fluorescent dyes, and approximating measurement spectra obtained by collecting detection values from the respective photodetectors using a linear sum of single staining spectra obtained in the microparticles individually labeled with the respective fluorescent dyes.

[Citation List]

[Patent Literature]

[PTL 1]
JP 2011-232259 A

WO 2019/031048 A1 provides an information processing device and method capable of easily calculating an error amount included in a measurement value. US 2012/056103 A1 relates to a fluorescence intensity control method for accurately computing the intensity of fluorescence emitted from each of a plurality of fluorochromes multiply-labeled on a microparticle.

US 2016/025621 A1 is directed to techniques for selecting antibody-dye conjugate combinations for use in flow cytometry.

US 2010/012853 A1 relates to a method of identifying important dye-detector spectral overlaps for use in designing flow cytometry experiments.

Non-patent document "Optimisation of multiplex immunofluorescence for a non-spectral fluorescence scanning system" (Chidozie C. Anyaegbu et al) describes the optimisation process and key considerations in development a multiplex fluorescence assay, and discusses some of the advantages and disadvantages of using multiplex IF with a non-spectral imaging system.

[Summary]

[Technical Problem]

Expression of various antigens can be evaluated simultaneously by unmixing processing of the fluorescence spectrum. However, performance of the unmixing processing may deteriorate depending on a combination of fluorescent markers. On the other hand, examination for selecting a better combination of fluorescent markers is often burdensome, for example, for researchers investigating expression of antigens. Therefore, an object of the present technology is to provide a technology for automatically presenting a better combination of fluorescent markers.

[Solution to Problem]

The invention is defined by the appended claims. The present technology provides an information processing device included by an information processing system, the information processing device including: a calculation processing unit configured to calculate an objective function regarding a combination of a biomolecule, the biomolecule being a labeling target in a sample, with a labeling phosphor, on the basis of first data listing information on the biomolecule, and second data listing information on the labeling phosphor; and an assignment information output unit configured to output information on assignment of the labeling phosphors to respective biomolecules, the information being generated on the basis of combination information acquired from a combinatorial optimization processing unit on the basis of a coefficient of the objective function.

The information processing device may acquire the first data input by a user. The information processing system may further include a database configured to hold the second data, and

the information processing device may acquire the second data by referring to the database on the basis of the first data.

The information processing device may acquire identification information of an algorithm for acquiring the assignment information, and

the algorithm may include at least a combinatorial optimization algorithm executed by the combinatorial optimization processing unit.

The objective function may be an objective function formulated as a constrained optimum selection problem of basis vector candidates.

A candidate for the basis vector selected in the optimum selection problem may correspond to a labeling phosphor that labels the biomolecule that is a labeling target.

The objective function can be an objective function of a binary variable in a quadratic format.

The objective function may be configured to indicate linear independence between the fluorescence spectra of the labeling phosphors assigned to the respective biomolecule.

The objective function may be configured to include a constraint condition regarding the number of phosphors assigned to one biomolecule.

The objective function may be configured to include a constraint condition that one phosphor is assigned to one biomolecule.

The objective function may be configured to include a constraint condition regarding fluorescence intensity regarding a combination of a biomolecule and a phosphor assigned to the biomolecule.

The fluorescence intensity may be based on an amount of the biomolecule and the fluorescence intensity of the phosphor assigned to the biomolecule.

The objective function may be configured to include dispersion of the fluorescence intensity information.

The information processing device may include a communication device capable of transmitting the coefficient of the objective function obtained by the calculation processing to an Ising machine specialized in optimization of a binary quadratic variable, and receiving a value of a binary variable selected by the Ising machine.

The Ising machine may have a function of selecting one or more combinations of spin variables corresponding to binary variables on the basis of the coefficient of the objective function.

The information processing device can acquire data regarding the combination of the biomolecule and the phosphor on the basis of the value of the spin variable acquired by the Ising machine.

The information processing device may acquire data regarding a combination of a biomolecule and a phosphor by converting the spin variable into a binary variable.

The biomolecule may be an antigen or an antibody.

Further, the present technology provides an information processing method including:

a calculation processing step of calculating an objective function regarding a combination of a biomolecule, the biomolecule being a labeling target in a sample,
and a labeling phosphor on the basis of first data listing information on the biomolecule and second data listing information on a fluorescence signal of the labeling phosphor; and
an output step of outputting information on assignment of the labeling phosphors to respective biomolecules, the information being generated on the basis of combination information acquired from a combinatorial optimization processing unit on the basis of a coefficient of the objective function.

Further, the present technology provides a program for causing an information processing device to execute:

a calculation processing step of calculating an objective function regarding a combination of a biomolecule, the biomolecule being a labeling target in a sample,

and a labeling phosphor on the basis of first data listing information on the biomolecule and second data listing information on a fluorescence signal of the labeling phosphor; and

an output step of outputting information on assignment of the labeling phosphors to respective biomolecules, the information being generated on the basis of combination information acquired from a combinatorial optimization processing unit on the basis of a coefficient of the objective function.

Further, the present technology provides an information processing system comprising the information processing device. This information processing system may further comprise a database being configured to hold the second data.

[Brief Description of Drawings]

[Fig. 1]

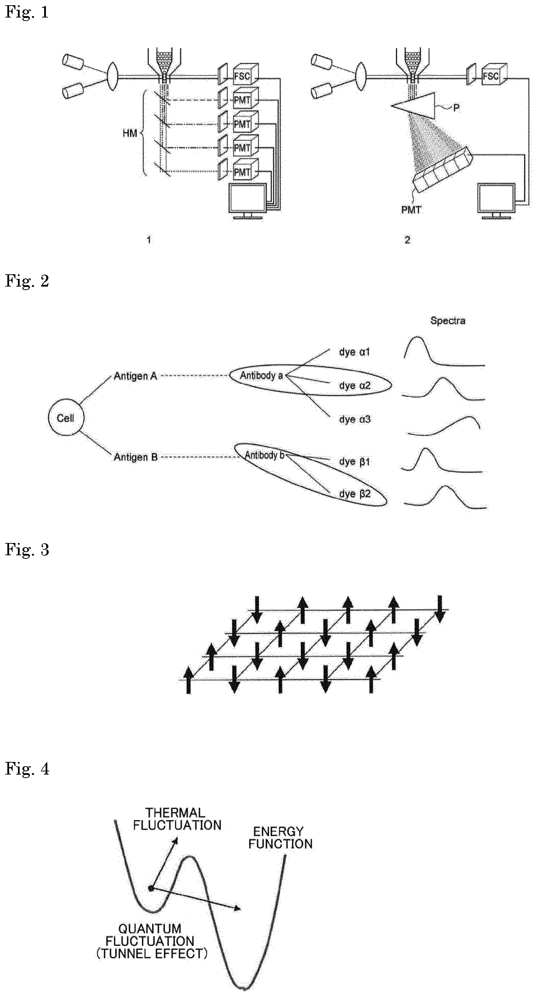

Fig. 1 is a schematic diagram of a configuration of filter type and spectral type flow cytometers.
[Fig. 2]
Fig. 2 is a diagram illustrating performance deterioration of unmixing processing.
[Fig. 3]
Fig. 3 is a schematic diagram of an Ising model.
[Fig. 4]
Fig. 4 is a diagram illustrating thermal fluctuation and quantum fluctuation.
[Fig. 5]

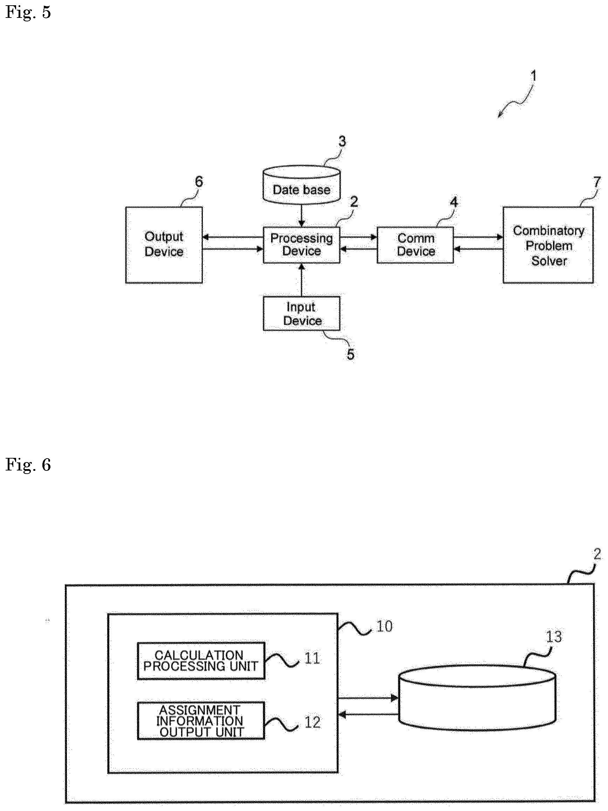

Fig. 5 is a block diagram of an example of an information processing system according to the present technology.
[Fig. 6]
Fig. 6 is a block diagram of an example of an information processing device included in the information processing system according to the present technology.
[Fig. 7]

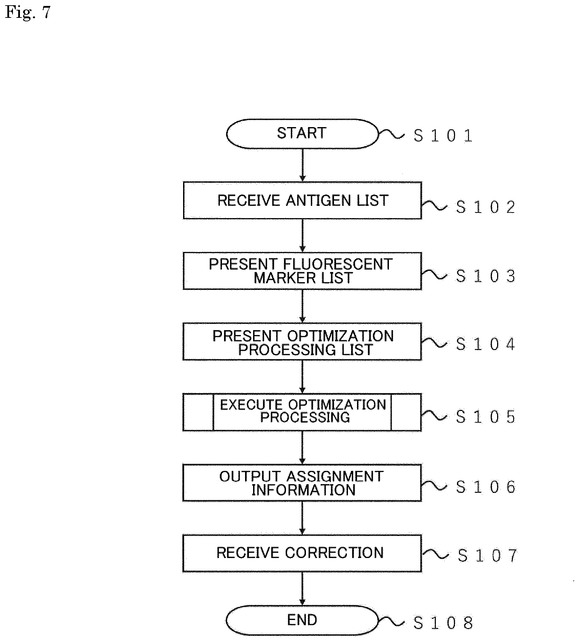

Fig. 7 is a flowchart of an information processing method according to the present technology.
[Fig. 8]

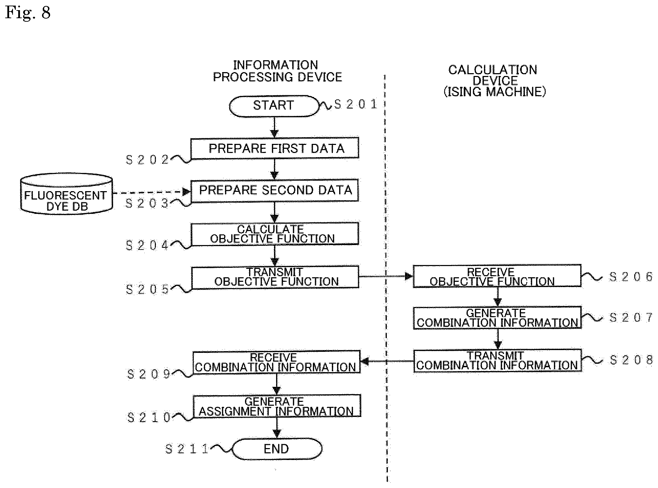

Fig. 8 is a flowchart of an optimization processing that is performed in an information processing method according to the present technology.

[Description of Embodiments]

Hereinafter, suitable embodiments for implementing the present technology will be described. Embodiments to be described below show typical embodiments of the present technology, and the scope of the present technology is not limited to these embodiments. The technology will be described in the following order.

1. First Embodiment (Information Processing System)

(1) Description of First Embodiment
(2) First Example of First embodiment
(2-1) Configuration Example of Information Processing System of Present Technology
(2-2) Example of Information Processing System of Present Technology configured as Particle Analysis System
(2-3) Example of Processing in Information Processing System of Present Technology

2. Second Embodiment (Information Processing Method)
3. Third Embodiment (Program)
4. Fourth Embodiment (Information Processing Device)
5. Fifth Embodiment (Calculation Device)

1. First Embodiment (Information Processing System)

(1) Description of First Embodiment

An information processing system according to the present technology includes an information processing device, the information processing device including: a calculation processing unit configured to calculate an objective function regarding a combination of a biomolecule, the biomolecule being a labeling target in a sample, with a labeling phosphor, on the basis of first data listing information on the biomolecule, and second data listing information on the labeling phosphor; and an assignment information output unit configured to output information on assignment of the labeling phosphors to respective biomolecules, the information being generated on the basis of combination information acquired from a combinatorial optimization processing unit on the basis of a coefficient of the objective function With the calculation processing unit and the assignment information output unit, it is possible to automatically select a better combination of the biomolecule and the labeling phosphor. Further, the calculation processing unit and the assignment information output unit can prevent, for example, deterioration in the performance of the unmixing processing.

A basic concept of the present technology will be described in detail below.

As described above, flow cytometers can be roughly classified into a filter type and a spectral type, for example, from the viewpoint of an optical system for fluorescence measurement. These two types of flow cytometers will be described with reference to Fig. 1.

For example, as shown in 1 of Fig. 1, a filter-type flow cytometer branches fluorescence into a plurality using a plurality of half mirrors HM, and performs measurement using, for example, a photomultiplier tube PMT through different filters. For example, the filter type also acquires a spectrum in a broad sense. However, in the filter type, it is necessary to prepare an optical system of the half mirror for each wavelength, and there is a problem that it is difficult to increase the number of channels due to a constraint of an optical arrangement. On the other hand, as illustrated in 2 of Fig. 1, one feature of the spectral type flow cytometer is that the fluorescence is acquired as a spectral spectrum by using a prism spectroscopic optical element P. Specifically, spectral fluorescence is acquired as multidimensional (multichannel) data using a photomultiplier tube PMT prepared for each wavelength. The spectral type is suitable for an increase in the number of channels in principle because the spectroscopy itself is easy. The spectral type has an advantage that the fluorescence can be analyzed in detail because the spectral spectrum can be measured in multiple channels.

Because the spectral type flow cytometer can analyze the fluorescence spectrum in detail as described above, the spectral type flow cytometer can increase the types of fluorescent markers and simultaneously inspect expression of a large number of different antigens. However, an actually obtained fluorescence spectrum includes mixed fluorescence of different fluorescent markers. Therefore, in order to evaluate the fluorescence of each fluorescent marker, it is necessary to decompose (separate) mixed fluorescence. This decomposition processing is called unmixing processing. The most commonly used scheme in the unmixing processing is a (weighted) least square method. In this method, the actually obtained fluorescence spectra are regarded as a linear sum of the fluorescence spectra of the dyes of the respective fluorescent markers, and a coefficient with the smallest (weighted) squared error is set as an amount of fluorescent marker bound to the antigen.

In the present specification, "fluorescent marker" means an antibody labeled with a phosphor, and is also referred to as, for example, a fluorescence-labeled antibody.

The unmixing processing can be described, for example, as follows.

It is assumed that the fluorescence spectrum is obtained from one of the cells by the flow cytometer as shown in Equation 1 below.

[Math. 1]

$$t = (t_1 \ldots t_N)^T \qquad \cdots \quad (1)$$

Here, N is the number of channels of the flow cytometer. On the other hand, it is assumed that there are M types of fluorescent markers, and a fluorescence spectrum of the m-th marker is obtained in advance as shown in Equation 2 below.

[Math. 2]

$$f^{(m)} = \left( f_1^{(m)} \ldots f_N^{(m)} \right)^T \qquad \cdots \quad (2)$$

Further, these fluorescence spectra are arranged to form a matrix of Equation 3 below.

[Math. 3]

$$F = \left( f^{(1)} \ldots f^{(M)} \right) \qquad \cdots \quad (3)$$

This matrix F is called a spillover matrix. Here, an amount to be obtained is an amount of a fluorescent marker bound to an antigen on a cell. An amount of an i-th fluorescent marker is $x_i$, and these are arranged side by side, and a vector as shown in Equation 4 below is obtained.

[Math. 4]

$$x = \left( x_1 \ldots x_M \right)^T \qquad \cdots \quad (4)$$

In the unmixing processing, an amount x of the fluorescent marker is obtained as a solution of a weighted least square method, as shown in Equation 5 below.

[Math. 5]

$$x^* = \arg \min_x \sum_{k=1}^{N} w_k \left( t_k - (Fx)_k \right)^2 \qquad \cdots \quad (5)$$

However, $w_k$ indicates a weight of a squared error of a k-th channel and is determined by a magnitude of noise in this channel.

A solution x* of this weighted least square method can be obtained analytically, as shown in Equation 6 below.

[Math. 6]

$$x^* = (F^T W F)^{-1} F^T W t \qquad \cdots \quad (6)$$

Here, w is a matrix in which weights are arranged as diagonal components, and is as shown in Equation 7 below.

[Math. 7]

$$W = \mathrm{diag}(w) \qquad \cdots \quad (7)$$

From the above, it is possible to calculate an amount of each fluorescent marker bound to the antigen through the unmixing processing of the fluorescence spectrum, and evaluate the expression of various antigens at the same time.

However, the performance of the unmixing processing can deteriorate, for example, in the following cases.

- A case in which the fluorescence spectra of two or more different fluorescent markers among the adopted fluorescent markers are the same or similar, and
- A case in which a combination of the fluorescence spectra of two or more different fluorescent markers among the adopted fluorescent markers are the same as or similar to spectra of other fluorescent markers.

A case in which, for a cell Cell expressing antigens A and B, an antibody a labeled with a fluorescent dye α2 is adopted as a fluorescent marker bound to the antigen A, and an antibody b labeled with a fluorescent dye β2 is adopted as a fluorescent marker bound to the antigen B, for example, as shown in Fig. 2 is assumed. In this case, because fluorescence spectra of the fluorescent dye α2 and the fluorescent dye β2 are similar, the performance of the unmixing processing may deteriorate.

Selection of a combination of fluorescent markers, that is, selection of a combination of the antibody and the antibody labeling phosphor, is examined in order to prevent the deterioration in the performance of the unmixing processing. For example, a plurality of types of antibodies labeled with different phosphors are prepared for each antigen, and a better fluorescent marker is selected from among a plurality of types of fluorescent markers to prevent the deterioration in the performance of the unmixing processing. More specifically, a user first specifies a plurality of antigens to be inspected and then selects a fluorescent marker to bind to each antigen, and this selection can be performed not to correspond to the case described above.

For measurement in a device or system that performs measurement using a combination of an antibody and a phosphor, such as a flow cytometer, it is conceivable to examine the following two things in order to assign a fluorescent marker with good fluorescence separation performance to each of a plurality of antigens that are measurement targets.

(i) Providing an index for evaluating what kind of combination is good
(ii) Providing a mechanism for searching for a large number of combinations at a high speed

Regarding (i) above, it is conceivable to adopt, for example, a correlation between fluorescence spectra as an index. More specifically, it is conceivable to calculate a correlation between the fluorescence spectra and combine phosphors in order from the phosphor with the smallest correlation so that two or more phosphors having the same or similar fluorescence spectra are not adopted. However, in this scheme, as combination processing progresses, highly correlated combinations remain, and overall optimization may not be achieved in the end. Further, with this scheme, it may not be possible to avoid a case in which a combination of spectra of two or more different phosphors described above is the same as or similar to spectra of other phosphors.

For (ii) above, a need for examination increases as the types of antigens and types of fluorescent markers increase. More specifically, when the number of types of antigens and/or the number of types of fluorescent markers per type of antigen to be evaluated at the same time increases, the number of combinations of fluorescent markers that can be adopted increases sharply. For example, when the number of antigen types is M and the number of fluorescent marker types per antigen is K, a good combination must be obtained from $M^K$ types of combinations. It is often required to evaluate more types of antigens at the same time, and the number of types of fluorescent markers per antigen is also increasing. Therefore, the number of combinations to be examined increases exponentially.

As an index regarding the performance of the unmixing processing, the present inventors focused on a determinant in Equation 8 below regarding the matrix F in which fluorescence spectra of phosphors are arranged.
[Math. 8]

$$\det(F^T F) \qquad \cdots \quad (8)$$

It is considered that the performance of the unmixing processing can be improved by adopting a combination that maximizes the determinant in Equation 8 above. Hereinafter, the determinant will be described in more detail.

When the two cases in which the performance of the unmixing processing may deteriorate are considered, it is conceivable that a condition of "a combination of the fluorescence spectra of two or more different fluorescent markers is the same as or similar to spectra of other fluorescent markers" should be satisfied in order to prevent the deterioration in the performance of the unmixing processing. This condition means that the fluorescence spectra of all the fluorescent markers are primarily independent. Primary independence of a vector is generally indicated by a determinant of a matrix in which vectors are arranged not being zero. However, because the spillover matrix F of Equation 3, which is a matrix in which the fluorescence spectra of the fluorescent markers described above are arranged, is not a square matrix, the determinant cannot be calculated. Therefore, the determinant in Equation 8 above, which is a determinant of a square matrix of Equation 9 below,
[Math. 9]

$$F^T F \qquad \cdots \quad (9)$$

is considered instead of the spillover matrix F.

Hereinafter, when a determinant $\det(F^T F)$ of Equation 8 above is not 0, this shows that the fluorescence spectra of the fluorescent markers are primarily independent.

First, when the fluorescence spectrum is a primary subordination,
[Math. 10]

$$\det(F^T F) = 0 \qquad \cdots \quad (10)$$

is proven as shown in Equation 10 above.

The fact that the fluorescence spectrum is a primary subordination means that a k-th fluorescence spectrum $f_k$ can be written as a linear combination of other spectra $f_m$. That is, $f_k$ and $f_m$ satisfy a relationship shown in Equation 11 below.
[Math. 11]

$$f_k = \sum_{m \neq k} c_m f_m \qquad \cdots (11)$$

Accordingly, the square matrix $F^TF$ of Equation 9 above can be written as shown in Equation 12 below.
[Math. 12]

$$F^TF = \begin{pmatrix} f_1 \cdot f_1 & \cdots & f_1 \cdot \sum_{m \neq k} c_m f_m & \cdots \\ f_2 \cdot f_1 & \cdots & f_2 \cdot \sum_{m \neq k} c_m f_m & \cdots \\ \cdots & \cdots & \cdots & \cdots \\ f_M \cdot f_1 & \cdots & f_M \cdot \sum_{m \neq k} c_m f_m & \cdots \end{pmatrix} = \begin{pmatrix} \begin{pmatrix} f_1 \cdot f_1 \\ f_2 \cdot f_1 \\ \cdots \\ f_M \cdot f_1 \end{pmatrix} & \cdots & \sum_{m \neq k} c_m \begin{pmatrix} f_1 \cdot f_m \\ f_2 \cdot f_m \\ \cdots \\ f_M \cdot f_m \end{pmatrix} & \cdots \end{pmatrix} \qquad \cdots (12)$$

As shown in Equation 12 above, the square matrix $F^TF$ is a primary subordination because a k-th column is written as a linear combination of the other columns. Therefore, this is as shown in Equation 10 above.
Next, it will be proved that, when the determinant $det(F^TF)$ in Equation 8 is 0 as shown in Equation 10 above, the matrix is a primary subordination.
When the relationship in Equation 10 above is satisfied, the k-th column of $F^TF$ may be written as a linear combination of other columns. Accordingly, this yields Equation 13 below.
[Math. 13]

$$F^TF = \begin{pmatrix} \begin{pmatrix} f_1 \cdot f_1 \\ f_2 \cdot f_1 \\ \cdots \\ f_M \cdot f_1 \end{pmatrix} & \cdots & \sum_{m \neq k} c_m \begin{pmatrix} f_1 \cdot f_m \\ f_2 \cdot f_m \\ \cdots \\ f_M \cdot f_m \end{pmatrix} & \cdots \end{pmatrix} = \begin{pmatrix} f_1^T \\ f_2^T \\ \cdots \\ f_M^T \end{pmatrix} \begin{pmatrix} f_1 & \cdots & \sum_{m \neq k} c_m f_m & \cdots & f_M \end{pmatrix} \qquad \cdots (13)$$

In Equation 13 above, because a k-th spectrum $f_k$ is written as a linear combination of the other spectra $f_m$, the square matrix $F^TF$ is a primary subordination.
From the above, it is understood that a condition that the spectrum of the fluorescent marker is primarily independent is
[Math. 14]

$$det(F^TF) \neq 0 \qquad \cdots (14)$$

Next, it is shown that, when the determinant $det(F^TF)$ in Equation 8 above is larger, the performance of decomposition tends to be good.
First, it is shown that the determinant $det(F^TF)$ in Equation 8 above is equal to or larger than 0 because the matrix $F^TF$ is a semi-positive definite matrix.
First, it is proved that the matrix $F^TF$ is the semi-positive definite matrix. When $F^TF$ is the semi-positive definite matrix, Equation 15 below is established due to the nature of the semi-positive definite matrix.
[Math. 15]

$$\forall x \in R^M, \quad x^T(F^TF)x \geqq 0 \qquad \cdots (15)$$

In Equation 15 above, when the left side is deformed, Equation 16 below is obtained.
[Math. 16]

$$x^T(F^TF)x = (Fx)^T(Fx) = Fx \cdot Fx \geq 0 \qquad \cdots \quad (16)$$

As shown in Equation 16 above, $F^TF$ is a semi-positive definite matrix.

Then, it is proved that the determinant of the semi-positive definite matrix is equal to or larger than 0.

From the characteristics of the semi-positive definite matrix, the following eigenvalues regarding the matrix $F^TF$

[Math. 17]

$$(\lambda_1 \ldots \lambda_M) \qquad \cdots \quad (17)$$

are all non-negative, that is, are as shown in Equation 18 below.

[Math. 18]

$$0 \leq \lambda_i \ (i = 1 \ldots M) \qquad \cdots \quad (18)$$

On the other hand, from a relationship between the determinant and the eigenvalues, a relationship as shown in Equation 19 below can be obtained.

[Math. 19]

$$\det(F^TF) = \prod_{i=1}^{M} \lambda_i \geq 0 \qquad \cdots \quad (19)$$

Therefore, the determinant in Equation 8 above has a value equal to or larger than 0.

It was shown above that the determinant in Equation 8 above is equal to or larger than 0.

Next, it will be shown below that the performance of the decomposition tends to be improved when a value of the determinant deviates from 0.

[Math. 20]

$$y = Fx + n \qquad \cdots \quad (20)$$

Modeling in this way makes it possible to express decomposition of the fluorescence spectrum as shown in Equation 21 below.

[Math. 21]

$$x' = x + (F^TF)^{-1}F^Tn \qquad \cdots \quad (21)$$

However, here, the weight matrix is an identity matrix.

First, a case in which there are only two fluorescent markers to be separated is considered. Further, for simplicity, it is assumed that the fluorescence spectrum is standardized by a squared norm. In this case, when the matrix $F^TF$ is calculated,

the matrix is expressed as Equation 22 below.

[Math. 22]

$$F^TF = \begin{pmatrix} f_1 \cdot f_1 & f_1 \cdot f_2 \\ f_2 \cdot f_1 & f_2 \cdot f_2 \end{pmatrix} \qquad \cdots \quad (22)$$

In Equation 22, when the spectrum is standardized, diagonal terms are all 1, and are as shown in Equation 23 below.

[Math. 23]

$$f_1 \cdot f_1 = f_2 \cdot f_2 = 1 \qquad \cdots \quad (23)$$

Further, in Equation 22 above, all off-diagonal terms are correlation coefficients r. That is, the matrix $F^TF$ is equivalent to a correlation matrix R as shown in Equation 24 below.
[Math. 24]

$$F^T F = R = \begin{pmatrix} 1 & r_{12} \\ r_{21} & 1 \end{pmatrix} = \begin{pmatrix} 1 & r \\ r & 1 \end{pmatrix} \qquad \cdots \quad (24)$$

Further, an inverse matrix and a determinant of the matrix $F^TF$ can be obtained as shown in Equations 25 and 26 below.
[Math. 25]

$$(F^T F)^{-1} = R^{-1} = \frac{1}{1 - r^2} \begin{pmatrix} 1 & -r \\ -r & 1 \end{pmatrix} \qquad \cdots \quad (25)$$

[Math. 26]

$$\det(F^T F) = |R| = 1 - r^2 \qquad \cdots \quad (26)$$

Using these to calculate a noise component after unmixing is considered. Noise n is as shown in Equation 27 below
[Math. 27]

$$\boldsymbol{n} = n \begin{pmatrix} \epsilon_1 \\ \cdots \\ \epsilon_N \end{pmatrix} \qquad \cdots \quad (27)$$

, a random number in Equation 28 below, which is a standard normal distribution,
[Math. 28]

$$\epsilon_i (i = 1 \dots N) \qquad \cdots \quad (28)$$

is used for expression, and noise after unmixing is as shown in Equation 29 below.
[Math. 29]

$$(F^T F)^{-1} F^T \boldsymbol{n} = n \frac{1}{1 - r^2} \begin{pmatrix} 1 & -r \\ -r & 1 \end{pmatrix} \begin{pmatrix} f_{11} & \cdots & f_{1N} \\ f_{21} & \cdots & f_{2N} \end{pmatrix} \begin{pmatrix} \epsilon_1 \\ \cdots \\ \epsilon_N \end{pmatrix} \qquad \cdots \quad (29)$$

As described above, because the fluorescence spectrum is standardized by the squared norm, a random number of Equation 30 below of the standard normal distribution
[Math. 30]

$$\epsilon_1', \ \epsilon_2' \qquad \cdots \quad (30)$$

is newly used to obtain Equation 31 below.
[Math. 31]

$$= n \frac{1}{1 - r^2} \begin{pmatrix} 1 & -r \\ -r & 1 \end{pmatrix} \begin{pmatrix} \epsilon_1' \\ \epsilon_2' \end{pmatrix} \qquad \cdots \quad (31)$$

Further, a random number of Equation 32 below, which is a standard normal distribution,
[Math. 32]

$$\epsilon_1'', \quad \epsilon_2'' \qquad \qquad \cdots (32)$$

is newly used to obtain Equation 33 below.
[Math. 33]

$$= n\frac{\sqrt{1+r^2}}{1-r^2}\begin{pmatrix}\epsilon_1''\\\epsilon_2''\end{pmatrix} = n\frac{\sqrt{2-|R|}}{|R|}\begin{pmatrix}\epsilon_1''\\\epsilon_2''\end{pmatrix} \qquad \cdots (33)$$

This noise decreases monotonically with respect to Equation 34 below
[Math. 34]

$$|R| = \det(F^T F) \qquad \qquad \cdots (34)$$

, and reaches a minimum when a maximum value is 1. That is, when noise added to the fluorescence spectrum is Gaussian noise having the same intensity for each channel, the performance of the unmixing processing is better when the determinant in Equation 8 above is larger.

As described above, in a case in which there are only two fluorescent markers to be separated, it can be seen that the performance of the unmixing processing becomes better when the determinant in Equation 8 above is larger. Further, even in a case in which there are three or more fluorescent markers to be separated, the performance of the unmixing processing tends to become better when the determinant in (8) above is larger, and this can be shown, for example, by performing simulation.

As described above, a combination of the fluorescent markers expected to improve the performance of the unmixing processing is considered to be a combination that maximizes the determinant in Equation 8 above regarding the matrix F in which the fluorescence spectra are arranged. Here, it is expected that an algorithm called quantum annealing or a simulated annealing or simulated branching algorithm similar thereto will be effective for a problem of combinatorial optimization. For any of the algorithms, processing using the algorithm becomes possible by treating a combinatorial optimization problem as a binary quadratic format optimization problem.

The quantum annealing and the Ising machine will be described hereinafter.

First, the quantum annealing will be described.

The quantum annealing is known as a hardware system that executes combinatorial optimization using qubits using a quantum device. Here, the qubit is an information unit (bit) that can realize quantum superposition and quantum entanglement. The quantum superposition is a phenomenon in which two states (values) are held in parallel and probabilistically (quantum probability amplitude) with 1 bit (1Qbit), and the quantum entanglement is a phenomenon in which a correlation state that cannot be realized classically between 2 bits is realized.

Because the quantum annealing is a type of calculation using qubits, the quantum annealing is often confused with quantum calculation itself. However, the operation is quite different from a quantum computer that has been traditionally examined. However, because the calculation is still a calculation using qubits, this is often positioned as one quantum calculation scheme these days.

The calculation that can be realized by the quantum annealing is to obtain a solution of the combinatorial optimization problem for minimizing an objective function of a quadratic format of Equation 35 below.
[Math. 35]

$$L = -\sum_{i,j} J_{ij}\sigma_i\sigma_j + \sum_k h_k\sigma_k \qquad \cdots (35)$$

In Equation 35 above, $\sigma_i$, $\sigma_j$, and $\sigma_k$ are variables that take one of two values including 0 and 1 and are variables of which an optimal solution is desired to be obtained. Further, $J_{ij}$ and $h_k$ are parameters and are given from the outside. For convenience of description below, here, these parameters are parameters in a case in which $\sigma_i$ is $\pm$ 1 instead of 0 or 1, for convenience.

In the quantum annealing, first, a physical model called the Ising model is realized by using a quantum device and circuit. Fig. 3 illustrates a schematic diagram of the Ising model.

The Ising model is a physical model proposed as a simple model for explaining ferromagnetism of a magnet. In Fig. 3, each grid point indicates an electron spin in which only an upward or downward spin moment is observed.

In statistical physics, a behavior of a physical model is described using an energy function, but the energy function of this physical model is described as shown in Equation 36 below.

[Math. 36]

$$H_{\text{Ising}} = -\sum_{i,j} J_{ij} \sigma_i^z \sigma_j^z + \sum_k h_k \sigma_k^z \qquad \cdots (36)$$

$\sigma_i$, $\sigma_j$, and $\sigma_k$ are variables indicating whether the spin is upward (-1) or downward (1). Further, $J_{ij}$ is a binding energy that determines a magnitude of an interaction between spins, and $h_k$ is an external magnetic field component parallel to the spin. For binding energy between the spins, a model in which only binding energy between adjacent spins has a non-zero value has been adopted in a model for explaining ferromagnetism of a magnet, but here generalization is made so that binding energy between arbitrary spins has a non-zero value. Such a model is called a spin glass.

It is easy to understand that an energy function of the Ising model is equivalent to an objective function of combinatorial optimization. Details of variable conversion or the like of $\pm$ 1 and 0, 1 are omitted. Therefore, when an Ising model having the same parameter as the objective function of combinatorial optimization is artificially configured and a spin of a basis state (minimum energy state) of the energy function is measured, an origin of an idea of the quantum annealing is to obtain an optimum solution (not local optimization but global optimization).

The minimum energy state is realized by a stepwise adiabatic transition from a superposition state to a basis state (a state transition that is slow enough to be regarded as a steady state) with an ultra-low temperature. This process is called quantum annealing.

Specifically, first, initially, a lateral magnetic field (a horizontal magnetic field) in Equation 37 below for realizing a superposition state of all combinations is applied.

[Math. 37]

$$H_{\text{Quantum}} = \Gamma \sum_k \sigma_k^x \qquad \cdots (37)$$

As shown in Equation 38 below, a superposition term is loosened step by step,

[Math. 38]

$$H_{\text{QA}}(t) = \frac{t}{T} H_{\text{Ising}} + \left(1 - \frac{t}{T}\right) H_{\text{Quantum}} \qquad \cdots (38)$$

and the basis state is finally realized. In a next procedure, the spin is measured in the basis state and is used as an optimum solution.

This process is "slow" in an adiabatic process, but in a real machine, the entire process can be completed in microseconds.

Further, it is said that the process can be realized at a higher speed than simulated annealing because a tunnel effect due to quantum fluctuation is used, as compared with the simulated annealing using thermal fluctuation, as shown in Fig. 4, for example. Effects thereof have also been confirmed experimentally in specific problem settings.

Regarding a quantum device that realizes qubits, for example, a quantum device using ions, light, and single electrons (quantum dots) is known, but a currently most effective quantum device is a superconducting device. Extremely low temperature is required to realize superconductivity. For this reason, a huge cooling device has been conventionally required. However, with the development of a cloud technology these days, these cooling devices are installed on the server side so that only calculation results can be acquired on the edge side.

Further, when combinatorial optimization using an Ising model has come into the limelight with the realization of quantum annealing, a device that speeds up combinatorial optimization itself represented by the Ising model by using an existing digital circuit or a gate circuit without using quantum annealing, for example, has appeared. This can also be realized on the edge side because this can be realized by a digital circuit without using superconductivity.

The present inventors have found that a problem of optimum selection of fluorescent markers of the flow cytometer can be formulated as a problem of optimum selection of basis vector candidates. Now, it is assumed that there are L basis vector candidates and an i-th basis vector is indicated by $u_i$. Using these basis vector candidates, A matrix U in Equation 39 below is created.
[Math. 39]

$$U = (u_1 \ldots u_L) \qquad \cdot \cdot \cdot \ (39)$$

Subsequently, a value indicating whether or not to select an i-th basis vector $u_i$ is as shown in Equation 40 below.
[Math. 40]

$$b_i \in \{0,1\} \qquad \cdot \cdot \cdot \ (40)$$

That is, a binary value $b_i$ is 1 when the basis vector $u_i$ is selected, and is 0 otherwise.
A matrix in which $b_i$ are arranged in diagonal items is B of Equation 41 below.
[Math. 41]

$$B = diag(b_1 \ldots b_L) \qquad \cdot \cdot \cdot \ (41)$$

Constraints regarding how to select the basis vector are imposed on the binary value $b_i$. For example, when the number of selected basis vectors is M (M < L), the following constraints are imposed.
[Math. 42]

$$\sum_{i=1}^{L} b_i = M \qquad \cdot \cdot \cdot \ (42)$$

Further, when the number of basis vectors to be selected is M (M < L) and an m-th basis vector is one of the $M_m$ number of candidates in Equation 43 below
[Math. 43]

$$u_{i_m}(i_m = i_{m,1} .. i_{m,M_m}) \qquad \cdot \cdot \cdot \ (43)$$

, M constraints of Equation 44 below are imposed.
[Math. 44]

$$\sum_{i_m = i_{m1} .. i_{mM_m}} b_{i_m} = 1 \qquad \cdot \cdot \cdot \ (44)$$

When the matrices U and B of the Equations 39 and 41 above are used, the next matrix Q becomes a matrix configured of selected basis vectors or 0 vectors.
[Math. 45]

$$Q = UB = (b_1 u_1 ..) \qquad \cdot \cdot \cdot \ (45)$$

On the other hand, the following matrix configured of only the selected basis vectors is F in Equation 46 below, as before.
[Math. 46]

$$F = (u_{i_1} \ldots u_{i_M}) \qquad \cdot \cdot \cdot \ (46)$$

Here, Equation 47 below indicates a combination of numbers for M basis vector candidates selected as basis vectors among L basis vector candidates.
[Math. 47]

$$(i_1 \ldots i_M) \qquad \cdots \quad (47)$$

In the description so far, a good combination of basis vectors is a combination that maximizes the determinant in Equation 8 above. However, this determinant is difficult to be converted into a binary quadratic format because a variable $b_i$ indicating the selection of the basis vectors does not appear clearly. Therefore, for example, an attempt is made to maximize a determinant $Q^TQ$ of Equation 48 below instead of the determinant $F^TF$ in Equation 9.
[Math. 48]

$$Q^TQ = \begin{pmatrix} b_1 b_1 u_1 \cdot u_1 & \ldots & b_1 b_L u_1 \cdot u_L \\ \ldots & \ldots & \ldots \\ b_L b_1 u_L \cdot u_1 & \ldots & b_L b_L u_L \cdot u_L \end{pmatrix} \qquad \cdots \quad (48)$$

However, in reality, this is as shown in Equation 49 below.
[Math. 49]

$$\det Q^TQ = 0 \qquad \cdots \quad (49)$$

This is because in a matrix $Q^TQ$, components of the rows and columns of i, which are as shown in Equation 50 below, are all 0.
[Math. 50]

$$b_i = 0 \qquad \cdots \quad (50)$$

Therefore, for row i and column i in which all the components are 0, only diagonal components are replaced with 1. This operation can be realized by Equation 51 below.
[Math. 51]

$$A = I - B + Q^TQ \qquad \cdots \quad (51)$$

Thus, a relationship of Equation 52 below is established.
[Math. 52]

$$\det A = \det F^TF \qquad \cdots \quad (52)$$

This relationship can be seen by considering an adjugate matrix (a matrix excluding rows and columns) regarding diagonal components replaced with 1.
From the above, a problem of optimum selection of the basis vector is replaced with a maximization problem of Equation 53 below.
[Math. 53]

$$\det(I - B + Q^TQ) \qquad \cdots \quad (53)$$

That is, the problem of optimum selection of the basis vector is formulated as a combinatorial optimization problem of Equation 54 below.
[Math. 54]

$$b^* = \arg\min_b [-\det(I - B + Q^TQ)] \qquad \cdots \quad (54)$$

Subsequently, this combinatorial optimization problem is considered to be a combinatorial optimization problem of a binary quadratic format. When the matrix A is written down in a greater detail, Equation 55 below is obtained.
[Math. 55]

$$A = I - B + Q^T Q$$
$$= \begin{pmatrix} 1 & b_1 b_2 r_{12} & ... & b_1 b_L r_{1L} \\ b_2 b_1 r_{21} & 1 & ... & b_2 b_L r_{2L} \\ ... & ... & ... & ... \\ b_L b_1 r_{L1} & b_L b_2 r_{L2} & ... & 1 \end{pmatrix}$$
$$= I + R \qquad\qquad \cdots \ (55)$$

Here,
[Math. 56]

$$r_{ij} = u_i \cdot u_j \qquad\qquad \cdots \ (56)$$

is a correlation coefficient between basis vectors $u_i$ and $u_j$. Further, R is a matrix defined by the variable b to be optimized, which is expressed by Equation 57 below.
[Math. 57]

$$R = R(b) = \begin{pmatrix} 0 & b_1 b_2 r_{12} & ... & b_1 b_L r_{1L} \\ b_2 b_1 r_{21} & 0 & ... & b_2 b_L r_{2L} \\ ... & ... & ... & ... \\ b_L b_1 r_{L1} & b_L b_2 r_{L2} & ... & 0 \end{pmatrix} \qquad\qquad \cdots \ (57)$$

Using this, the combinatorial optimization problem can be converted as shown in Equation 58 below.
[Math. 58]

$$b^* = \arg\min_b \left[ -\det\left(I + R(b)\right) \right] \qquad\qquad \cdots \ (58)$$

In Equation 58, because the determinant still remains, it is necessary to write down the determinant. However, when the determinant is written as it is, the number of terms increases with an exponential function of a rank of the matrix, making the calculation difficult. Therefore, a Taylor expansion of the determinant as shown in Equation 59 below is considered. (Reference: https://qiita.com/research-PORT-INC/items/3261020c2a7f0a40e6c4)
[Math. 59]

$$f(x) = \det(I + Ax)$$
$$= f(0) + f^{(1)}(0)x + \frac{1}{2}f^{(2)}(0)x^2 + \frac{1}{3!}f^{(3)}(0)x^3 + \cdots \qquad\qquad \cdots \ (59)$$

For each differential coefficient, a first derivative is obtained according to a definition of differentiation as shown in Equation 60 below. (As described on the reference web page)
[Math. 60]

$$f'(x) = \lim_{\delta x \to 0} \frac{\det(I + A(x + \delta x)) - \det(I + Ax)}{\delta x}$$

$$= \lim_{\delta x \to 0} \frac{\det(I + Ax)\{\det(I + (I + Ax)^{-1}A\delta x) - 1\}}{\delta x}$$

$$= \lim_{\delta x \to 0} \frac{\det(I + Ax)\{1 + \text{tr}((I + Ax)^{-1}A)\delta x - 1\}}{\delta x}$$

$$= \det(I + Ax)\,\text{tr}((I + Ax)^{-1}A)$$

$$= f(x)\text{tr}(F^{-1}(x)) \qquad \cdots \quad (60)$$

Here, F(x) was defined as Equation 61 below.
[Math. 61]

$$F(x) = A^{-1}(I + Ax) \qquad \cdots \quad (61)$$

Similarly, when a second derivative is obtained, the second derivative is as shown in Equation 62 below.
[Math. 62]

$$f^{(2)}(x) = f'(x)\text{tr}(F^{-1}) - f(x)\text{tr}(F^{-2})$$

$$= f(x)\left(\text{tr}(F^{-1})\right)^2 - \text{tr}(F^{-2}) \qquad \cdots \quad (62)$$

Similarly, a third derivative is as shown in Equation 63 below.
[Math. 63]

$$f^{(3)}(x) = f'(x)\left(\left(\text{tr}(F^{-1})\right)^2 - \text{tr}(F^{-2})\right) + f(x)\left(-2\,\text{tr}(F^{-1})\text{tr}(F^{-2}) + 2tr(F^{-3})\right)$$

$$= f(x)\left(\left(\text{tr}(F^{-1})\right)^3 - 3\text{tr}(F^{-1})\text{tr}(F^{-2}) + 2\text{tr}(F^{-3})\right) \qquad \cdots \quad (63)$$

On the other hand, when x = 0 is substituted for f(x) and $F^{-1}(x)$, Equations 64 and 65 below are obtained.
[Math. 64]

$$f(0) = 0 \qquad \cdots \quad (64)$$

[Math. 65]

$$F^{-1}(0) = A \qquad \cdots \quad (65)$$

From the above, Equations 66, 67, and 68 below can be obtained.
[Math. 66]

$$f^{(1)}(0) = \text{tr}(A) \qquad \cdots \quad (66)$$

[Math. 67]

$$f^{(2)}(0) = \text{tr}(A)^2 - \text{tr}(A^2) \qquad \cdots \quad (67)$$

[Math. 68]

$$f^{(3)}(0) = \text{tr}(A)^3 - 3\text{tr}(A)\text{tr}(A^2) + 2\text{tr}(A^3) \qquad \cdots \ (68)$$

Using this result, the Taylor expansion of f(x) is written as shown in Equation 69 below. The above reference web page was referred to until Equation 69 is obtained.
[Math. 69]

$$f(x) = f(0) + f^{(1)}(0)x + \frac{1}{2!}f^{(2)}x^2 + \frac{1}{3!}f^{(3)}x^3 + \cdots$$

$$= 1 + \text{tr}(A)x + \frac{1}{2}\big(\text{tr}(A)^2 - \text{tr}(A^2)\big)x^2 + \frac{1}{6}\big(\text{tr}(A)^3 - 3\text{tr}(A)\text{tr}(A^2) + 2\text{tr}(A^3)\big)x^3 + \cdots \qquad \cdots \ (69)$$

Then,
[Math. 70]

$$A = \frac{R}{x} \qquad \cdots \ (70)$$

tr(A), tr($A^2$), and tr($A^3$) are calculated for an actual R as shown in Equations 71, 72, and 73 below.
[Math. 71]

$$\text{tr}(A) = 0 \qquad \cdots \ (71)$$

[Math. 72]

$$\text{tr}(A^2) = \frac{1}{x^2}\text{tr}(R^2) = \frac{1}{x^2}\sum_{i=1}^{L}\delta_{ij}\sum_{j=1}^{L}\sum_{k=1}^{L}(R)_{ik}(R)_{jk} = \frac{1}{x^2}\sum_{i=1}^{L}\sum_{k=1}^{L}b_i b_k r_{ik}^2 \qquad \cdots \ (72)$$

[Math. 73]

$$\text{tr}(A^3) = \frac{1}{x^3}\text{tr}(R^3) = \frac{1}{x^3}\sum_{i=1}^{L}\delta_{ij}\sum_{j=1}^{L}\sum_{k=1}^{L}\sum_{l=1}^{L}(R)_{ik}(R)_{kl}(R)_{lj} = \frac{1}{x^3}\sum_{i=1}^{L}\sum_{k=1}^{L}\sum_{l=1}^{L}b_i b_k b_l r_{ik}r_{il}r_{kl} \qquad \cdots \ (73)$$

As described above, Equation 74 below can be obtained.
[Math. 74]

$$\det F^T F = f(I + R) = 1 - \frac{1}{2}\sum_{i=1}^{L}\sum_{k=1}^{L}b_i b_k r_{ik}^2 + \frac{1}{3}\sum_{i=1}^{L}\sum_{k=1}^{L}\sum_{l=1}^{L}b_i b_k b_l r_{ik}r_{il}r_{kl} + \cdots \qquad \cdots \ (74)$$

That is, it can be said that an optimum selection problem of a basis vector candidate is a problem of minimizing the objective function of Equation 75 below.
[Math. 75]

$$L(\boldsymbol{b}) = -\left[1 - \frac{1}{2}\sum_{i=1}^{L}\sum_{k=1}^{L}b_i b_k r_{ik}^2 + \frac{1}{3}\sum_{i=1}^{L}\sum_{k=1}^{L}\sum_{l=1}^{L}b_i b_k b_l r_{ik}r_{il}r_{kl} + \cdots\right] \qquad \cdots \ (75)$$

Taking into account the above-described constraint regarding the basis vector, a combinatorial optimization equation is formulated as shown in Equation 76 below.
[Math. 76]

$$\boldsymbol{b}^* = \arg\min_{\boldsymbol{b}} \left[ L(b) + \sum_{m=1}^{M} \lambda_m \left( 1 - \sum_{i_m = i_{m1} \cdots i_{mM_m}} b_{i_m} \right)^2 \right] \qquad \cdots (76)$$

In Equation 76, $\lambda_m$ is a positive real parameter, and is a large value so that the second term is always satisfied, or a dynamically determined value.

A constraint term of a second term in Equation 76 is in a binary quadratic format, as can be easily understood. However, $L(b)$ in the first term has tertiary or higher terms, and does not have a binary quadratic format. Therefore, Equation 76 cannot be implemented in the Ising machine.

Therefore, here, $L(b)$ is approximated using up to the quadratic term. In this case, when the combinatorial optimization is formulated, the combinatorial optimization can be written as follows.
[Math. 77]

$$\boldsymbol{b}^* = \arg\min_{\boldsymbol{b}} \left[ \frac{1}{2} \sum_{i=1}^{L} \sum_{k=1}^{L} b_i b_k r_{ik}^2 + \sum_{m=1}^{M} \lambda_m \left( 1 - \sum_{i_m} b_{i_m} \right)^2 \right] \qquad \cdots (77)$$

As a derivative form that can be easily considered from Equation 77, instead of
[Math. 78]

$$r_{ik}^2 \qquad \cdots (78)$$

in Equation 77, a monotonous increase function of Equation 79 below
[Math. 79]

$$r_{ik} \geq 0 \qquad \cdots (79)$$

may be used. For example, this is Equation 80 below or the like.
[Math. 80]

$$(r_{ik})^n, 1 \leq n \qquad \cdots (80)$$

Hereinafter, when the conversion is advanced without changing Equation 78, this combinatorial optimization is in a binary quadratic format and can be easily implemented in the Ising machine. Specifically, Equation 81 below
[Math. 81]

$$\sigma_i = 1 - 2b_i \qquad \cdots (81)$$

is used for conversion, so that Equation 77 can be formulated as a combinatorial optimization problem as shown in Equation 82 below. Thus, the objective function formulated as the constrained optimum selection problem of the basis vector candidates can be used for selection of the fluorescent marker.
[Math. 82]

$$\sigma^* = \arg\min_{\sigma} \left[ \frac{1}{8} \sum_{i=1}^{L} \sum_{k=1}^{L} (1 - \sigma_i)(1 - \sigma_k) r_{ik}^2 + \sum_{m=1}^{M} \lambda_m \left( 1 - \frac{1}{2} \sum_{i_m} (1 - \sigma_{i_m}) \right)^2 \right] \qquad \cdots (82)$$

The objective function of Equation 82 above becomes Hamiltonian of the Ising model. A coefficient of the objective function of Equation 82 above can be easily obtained by using a program. Therefore, the spin can be optimized by substituting the obtained coefficient for the Ising machine. It is possible to obtain a solution of optimization by returning the obtained optimization spin to a binary variable as shown in Equation 83 below.

[Math. 83]

$$b_i^* = \frac{1 - \sigma_i^*}{2} \qquad \cdots \quad (83)$$

L(b) is approximated above for the actual R using terms up to a quadratic term, but it is also possible to perform approximation using terms up to a slightly higher dimension. Specifically, it is considered to reduce a tertiary term of Equation 84 below to a quadratic term.
[Math. 84]

$$\sum_{i=1}^{L} \sum_{k=1}^{L} \sum_{l=1}^{L} b_i b_k b_l r_{ik} r_{il} r_{kl} \qquad \cdots \quad (84)$$

First, Equation 85 below is obtained by using Equation 81 above.
[Math. 85]

$$\sum_{i=1}^{L} \sum_{k=1}^{L} \sum_{l=1}^{L} b_i b_k b_l r_{ik} r_{il} r_{kl} = \frac{1}{8} \sum_{i=1}^{L} \sum_{k=1}^{L} \sum_{l=1}^{L} (1 - \sigma_i)(1 - \sigma_k)(1 - \sigma_l) r_{ik} r_{il} r_{kl} \qquad \cdots \quad (85)$$

In Equation 85 above, Equation 86 below appears as a tertiary term regarding spin.
[Math. 86]

$$\sum_{i=1}^{L} \sum_{k=1}^{L} \sum_{l=1}^{L} \sigma_i \sigma_k \sigma_l r_{ik} r_{il} r_{kl} \qquad \cdots \quad (86)$$

An auxiliary spin of Equation 87 below are prepared in order to reduce this dimension.
[Math. 87]

$$\sigma_{kl} = \sigma_k \sigma_l \qquad \cdots \quad (87)$$

The dimension can be reduced to a quadratic term as shown in Equation 88 below by using the auxiliary spin of Equation 87 above.
[Math. 88]

$$\sum_{i=1}^{L} \sum_{k=1}^{L} \sum_{l=1}^{L} \sigma_i \sigma_k \sigma_l r_{ik} r_{il} r_{kl} = \sum_{i=1}^{L} \sum_{k=1}^{L} \sum_{l=1}^{L} \sigma_i \sigma_{kl} r_{ik} r_{il} r_{kl} \qquad \cdots \quad (88)$$

Regarding Equation 88 above, it is necessary to optimize Equation 89 below to 0 as a constraint for not causing mismatch between the auxiliary spin and an original spin.
[Math. 89]

$$H_{kl} = 3\sigma_{kl} + \sigma_k \sigma_l - 2\sigma_k \sigma_{kl} - 2\sigma_l \sigma_{kl} \qquad \cdots \quad (89)$$

This term is a constraint term added to the objective function, but because the term is a quadratic term of spin, the term can be implemented in the Ising machine. Primary and quadratic coefficients of the spin to be substituted for the Ising machine can be easily obtained by using a program. When the coefficients obtained by the program are substituted for the Ising machine, it is possible to optimize the spin in the Ising machine. The solution of the optimization can be obtained by returning the obtained optimization spin to a binary variable as shown in Equation 90 below.
[Math. 90]

$$b_i^* = \frac{1 - \sigma_i^*}{2} \qquad \cdots (90)$$

As described above, in order to automatically select a better combination of the biomolecule and the labeling phosphor, it is possible to use an objective function regarding the combination of the biomolecule and the labeling phosphor described above. It is possible to acquire the objective function, particularly, the coefficient of the objective function, for example, using a program, as described above. For example, with a general-purpose information processing device such as a computer owned by a user who selects the combination, it is possible to calculate the objective function, particularly, the coefficient of the objective function. The combinatorial optimization processing unit that performs the combinatorial optimization processing is caused to generate combination information by using the objective function, and the generated combination information is used, thereby making it possible for the general-purpose information processing device to generate information on assignment of the labeling phosphors to respective biomolecules.

(2) First Example of First Embodiment

(2-1) Configuration Example of Information Processing System of Present Technology

An information processing system according to the present technology will be described below with reference to Fig. 5. Fig. 5 illustrates a configuration example of the information processing system according to the present technology.
The information processing system 1 shown in Fig. 5 includes an information processing device 2, a database 3, a communication device 4, an input device 5, an output device 6, and a calculation device 7. The information processing system 1 may be a system for presenting, to the user, a combination of a biomolecule (particularly, an antigen or an antibody) used in a particle analysis device such as a flow cytometer and an antibody labeling phosphor.
A block diagram of the information processing device 2 is shown in Fig. 6. As shown in Fig. 6, the information processing device 2 includes a processing unit 10. The processing unit 10 includes a calculation processing unit 11 that calculates an objective function regarding a combination of a biomolecule and a labeling phosphor, and an assignment information output unit 12 that outputs the information on assignment of the labeling phosphors to respective biomolecules. The information processing device 2 may further include a storage unit 13.
The calculation processing unit 11 can calculate the objective function regarding a combination of the biomolecule with the labeling phosphor on the basis of the first data listing information on the biomolecule that is a labeling target of the sample and the second data listing information on the labeling phosphor.
The first data includes information on the biomolecule that is a labeling target of the sample. The first data may be data input by the user. The information on the biomolecule may be, for example, a name or abbreviation of the biomolecule, or a number for specifying the biomolecule (for example, an ID number). The biomolecule may be, for example, a biomolecule designated as a labeling target by the user. The biomolecule may be, for example, an antibody or an antigen. The sample may be, for example, a sample containing particles to be described below or may be, more particularly, biological particles, and even more particularly, a sample including cells. The biomolecule may be a cell surface marker.
The second data includes information on the labeling phosphor. The information preferably includes the fluorescence spectrum of each of the labeling phosphors. The information may be, for example, a name or abbreviation of the labeling phosphor, or a number (for example, an ID number) for specifying the labeling phosphor.
The objective function is preferably an objective function formulated as the constrained optimum selection problem of basis vector candidates. The candidate for the basis vector selected in the optimum selection problem may correspond to a labeling phosphor that labels the biomolecule that is a labeling target. For example, the candidate may correspond to a fluorescence spectrum of a phosphor that can be used as the labeling phosphor.
As described in "(1) Description of First Embodiment" above, in the unmixing processing, an actually obtained fluorescence spectrum is regarded as a linear sum of the fluorescence spectra of the dyes of the respective fluorescent markers. That is, the fluorescence spectra of the D channels (D frequencies) can be regarded as a D-dimensional vector (D can be an arbitrary positive number), and the actual fluorescence spectrum is a linear sum with the fluorescence spectrum of each of the labeling phosphors as a basis. Therefore, as described above, the labeling phosphor, particularly, the fluorescence spectrum of the labeling phosphor can be treated as a basis vector candidate in the constrained optimum selection problem.
The objective function is preferably an objective function of a binary variable in a quadratic format. Because the objective function is an objective function of a binary variable in a quadratic format, the objective function can be implemented in the Ising machine.

The objective function may be configured to indicate linear independence between the fluorescence spectra of the labeling phosphors assigned to the respective biomolecule. For example, the objective function may be an objective function on the basis of a determinant indicating the linear independence. The objective function based on the determinant indicating the linear independence may be an objective function based on the determinant $\det(F^TF)$ of Equation 8 described in "(1) Description of First Embodiment" above or a determinant equivalent thereto.

The objective function may preferably be configured to include a constraint condition regarding the number of phosphors assigned to one biomolecule, and may be, more preferably, configured to include a constraint condition that one phosphor is assigned to one biomolecule.

The objective function may be configured to include a constraint condition regarding fluorescence intensity regarding a combination of a biomolecule and a phosphor assigned to the biomolecule. The fluorescence intensity may be based on an amount of the biomolecule and the fluorescence intensity of the phosphor assigned to the biomolecule.

According to these constraints, for example, a brighter labeling phosphor can be assigned to a smaller amount of biomolecule and a darker labeling phosphor can be assigned to a higher amount of biomolecule.

The objective function may be, for example, an objective function obtained by converting Equation 76 described in "(1) Description of First Embodiment" above into a binary quadratic format. The objective function converted into the binary quadratic format is, for example, the objective function of Equation 82 described in "(1) Description of First Embodiment" above.

In a preferred embodiment of the present technology, the objective function may be configured to include dispersion of the fluorescence intensity. For example, the objective function may include a term regarding the dispersion of the fluorescence intensity. More specifically, the objective function may be based on, for example, the objective function in which the item regarding the dispersion of the fluorescence intensity has been added to Equation 76 described in "(1) Description of First Embodiment" above. An example of the item regarding the dispersion of the fluorescence intensity will be described below.

For example, it is considered that an assumed intensity of an k-th fluorescent marker is labeled as $a_k$. In this case, an intensity of the fluorescent marker with respect to the m-th antigen is expressed by Equation 91 below.

[Math. 91]

$$A_m = \sum_{i_m = i_{m1}..i_{mM_m}} a_{i_m} b_{i_m} \qquad \cdots (91)$$

Further, the dispersion of the fluorescence intensity is expressed by Equation 92 below.

[Math. 92]

$$V(b_{1...L}) = V[A] = \frac{1}{M} \sum_{m=1}^{M} \left( \sum_{i_m = i_{m1}..i_{mM_m}} a_{i_m} b_{i_m} \right)^2 - \left( \frac{1}{M} \sum_{m=1}^{M} \sum_{i_m = i_{m1}..i_{mM_m}} a_{i_m} b_{i_m} \right)^2 \qquad \cdots (92)$$

Equation 92 is a quadratic equation of b. By incorporating this dispersion of the fluorescence intensity into Equation 76 above, an objective function formulated as shown in Equation 93 below can be obtained. The objective function of Equation 93 may be adopted in the present technology.

[Math. 93]

$$b^* = \arg\min_b \left[ L(b) + \lambda V(b) + \sum_{m=1}^{M} \lambda_m \left( 1 - \sum_{i_m = i_{m1}..i_{mM_m}} b_{i_m} \right)^2 \right] \qquad \cdots (93)$$

Fluorescence intensity of the selected fluorescent marker (for example, a peak value or a squared norm) can be aligned by the objective function incorporating the dispersion of the fluorescence intensity. The fluorescence intensity may be based on, for example, the fluorescence intensity of the phosphor included in the fluorescent marker and the amount of molecules detected by the fluorescent marker (for example, an amount of antigen, particularly, an amount of expression of antigens). For example, the amount of the molecule may be, for example, an amount divided into a plurality of stages (for example, stages 2 to 10, particularly, stages 2 to 8, and more particularly, stages 2 to 5), and more specifically, an amount divided into three stages including "large", "medium", and "small". Similar to the amount, the fluorescence intensity of the phosphor may be fluorescence intensity divided into a plurality of stages (for example, stages 2 to 10, particularly, stages 2 to 8, and more particularly, stages 2 to 5), and more specifically, a degree of brightness divided into three stages including "bright", "medium", and "dark". The number of these stages may be set by a user, such as a researcher.

The assignment information output unit 12 can output information on assignment of the labeling phosphors to respective biomolecules on the basis of the combination information acquired from the combinatorial optimization processing unit on the basis of the coefficient of the objective function. As will be described below, the optimization processing unit acquires the combination information on the basis of the coefficient of the objective function. The assignment information output unit 12 can generate information on the assignment of the labeling phosphor to each biomolecule on the basis of the combination information.

The combination information may include, for example, a spin variable. The spin variable may be preferably a spin variable optimized by the optimization processing unit. The assignment information output unit 12 can generate, preferably, the information on the assignment of the labeling phosphor to each biomolecule on the basis of a value of the spin variable. For example, the assignment information output unit may acquire the information on the assignment of the labeling phosphor to each biomolecule, particularly, data regarding the combination of the biomolecule and the phosphor, by converting the spin variable into a binary variable. The conversion of the spin variable to the binary variable may be performed, for example, as described with reference to Equation 90 in "(1) Description of First Embodiment" above.

The assignment information output unit 12 outputs the generated assignment information. For example, the assignment information output unit 12 can output the assignment information to the output device 6 connected to the information processing device 2. When the output device 6 is a display device, the display device may display the assignment information so that the user can visually recognize the assignment information.

The storage unit 13 may be configured to store various types of data that are used in the present technology. A configuration of the storage unit 13 may be appropriately selected by those skilled in the art. For example, the storage unit 13 can store the first data, the second data, the objective function, the combination information, and the assignment information.

A configuration example of the information processing device 2 will be described hereinafter. The processing in the processing unit 10 of the information processing device 2 can be realized by, for example, the following configuration. The information processing device 2 may include, for example, a central processing unit (CPU), a RAM, and a ROM. The CPU, RAM, and ROM may be connected to each other via a bus. An input and output interface may be further connected to the bus. The communication device 4, the input device 5, and the output device 6 may be connected to the bus via the input and output interface. The information processing device 2 may be configured of, for example, a general-purpose computer.

The database 3 may store information that is used as the second data by the information processing device 2, and has, for example, the second data listing the information on the labeling phosphor. For example, in the database 3, the information is as described above. The database 3 may be provided inside the information processing device 2 or may exist outside the information processing device 2, and in this case, the database 3 may be connected to the information processing device 2 by wire or wirelessly.

The information processing device 2 can refer to the database 3 on the basis of the first data, for example, and acquire the second data from the database 3. For example, the information processing device 2 can acquire the information on the labeling phosphor associated with each of the biomolecule groups listed in the first data. The acquired information on the labeling phosphor can be used by the calculation processing unit.

The database 3 may transmit the second data to the information processing device 2 in response to reception of the first data from the information processing device 2, for example. The database 3 selects, for example, a labelable labeling phosphor for each of the biomolecules included in the first data, and creates a phosphor list. That is, a plurality of phosphor lists are generated. The database 3 transmits the plurality of phosphor lists to the information processing device 2. The plurality of phosphor lists can be used as the second data by the information processing device 2.

The communication device 4 transmits the objective function calculated by the information processing device 2 (particularly, the calculation processing unit 11) to the calculation device 7 (particularly, the optimization processing unit included in the calculation device 7). For example, when the Ising machine is used as the calculation device 7, the communication device 4 may be configured, for example, so that the coefficient of the objective function obtained by the calculation processing unit 11 can be transmitted to the Ising machine. Further, the communication device 4 may be configured to be able to receive a value of the binary variable selected by the Ising machine.

The communication device 4 may connect the information processing device 2 to the network by wire or wirelessly. The information processing device 2 may be connected to the calculation device 7 via, for example, a network.

The communication device 4 may be provided inside the information processing device 2 or may be provided outside the information processing device 2. A configuration of the communication device 4 may be appropriately selected by those skilled in the art.

The input device 5 may be configured to be able to receive measurement data of the sample, for example, fluorescence measurement data of the sample. In an embodiment, the data acquired by a particle analysis device such as a flow cytometer, particularly, optical data may be configured to be able to be received. The input device 5 may include an interface capable of receiving the data.

Further, the input device 5 may include an interface configured to be able to receive an input of various types of data. For example, the input device 5 may be configured to receive a biomolecule selection operation of the user. The input device 5 may include, for example, a mouse, a keyboard, and a touch panel as devices that receive such an operation.

The output device 6 may be configured to be able to output various types of data. In an embodiment, the output device 6 includes a display device.

The display device may display, for example, an image for requesting the user to select a biomolecule included in the first data. The display device may display, for example, a list of biomolecules included in the first data.

The display device may display, for example, a list of labeling phosphors included in the second data. Further, the display device may display the fluorescence spectrum of the labeling phosphor.

In a preferred embodiment of the present technology, the display device may display a screen for requesting the user to select an optimization scheme for optimizing the combination of the biomolecule and the labeling phosphor. The screen may be a screen on which one or a plurality of optimization schemes can be selected from among a plurality of optimization schemes. An optimization scheme based on the objective function may be included as one of the optimization schemes that can be selected on the screen.

The display device can display the assignment information output by the assignment information output unit 12. More specifically, it is possible to display a combination of the biomolecule and the labeling phosphor.

The calculation device 7 includes a combinatorial optimization processing unit that generates combination information on the combination of the biomolecule and the labeling phosphor on the basis of the coefficient of the objective function. The calculation device including the combinatorial optimization processing unit is also called a combinatorial optimization solver. The calculation device 7 including the combinatorial optimization processing unit may be configured as, for example, the Ising machine, and more specifically, may be configured as an Ising machine that optimizes a binary quadratic variable. The calculation device 7 may be configured as, for example, a semiconductor CMOS circuit, a digital circuit, a GPU, or an FPGA.

The Ising machine has a function of selecting one or more combinations of spin variables on the basis of the coefficient of the objective function, and has, more specifically, a function of selecting one or more combinations of spin variables corresponding to a binary variable on the basis of the coefficient of the objective function.

The calculation device 7 may be an Ising machine that solves a multivariable combinatorial optimization problem. For example, the calculation device 7 can replace a problem of a calculation target with an Ising model (also referred to as Quadratic Unconstrained Binary Optimization (QUBO)) that is a model indicating a behavior of a spin of a magnetic material, and perform the calculation. The calculation device 7 may be an Ising machine that calculates a combination of values of variables with which the value of the objective function is minimized by performing the simulated annealing using a digital circuit, or may be an Ising machine that performs the same calculation by performing quantum annealing using a superconducting circuit. Examples of the calculation device 7 can include an Ising machine that reproduces an operation of the Ising model with a semiconductor CMOS circuit, an Ising machine configured of a digital circuit inspired by a quantum phenomenon, and an Ising machine in which a simulated branch algorithm is realized by an FPGA. With such an Ising machine, a function of the combinatorial optimization processing unit can be realized.

(2-2) Example of Information Processing System of Present Technology Configured as Particle Analysis System

In an embodiment of the present technology, the information processing system of the present technology may be configured as a particle analysis system. The particle analysis system may be configured, for example, as a system that performs flow cytometry or may be configured as a system that performs cell sorting. The particle analysis system may include, for example, a light irradiation unit that irradiates particles as analysis targets with light, a chip provided with a flow path through which the particles (particularly, cells) flow, and a detection unit that detects the light generated by the light irradiation, in addition to a configuration of the information processing device and the calculation device described above. For example, the light irradiation unit and the detection unit may be configured as one particle analysis device, and the particle analysis device may be combined with the information processing device and configured as the particle analysis system. The particle analysis device may be connected to the information processing device by wire or wirelessly, or may be connected via a network.

The light irradiation unit is configured to irradiate a predetermined position in the flow path of the chip with the light. When the particles pass through the light irradiation position in the flow path, the particles are irradiated with the light, and as a result, fluorescence is generated. That is, the light can act, as excitation light, on the particles, particularly, the phosphor labeling the particles. The light irradiation unit may include, for example, a laser light source that emits laser light (excitation light) to the particles. A configuration of the light irradiation unit may be appropriately selected by those skilled in the art.

The chip may be configured as, for example, a flow cell. The chip is provided with a flow path. A flow path structure provided in the chip, for example, is configured to form a flow (particularly, a laminar flow) in which particles flow in substantially a row. As the flow path structure provided in the chip, for example, a flow path structure known in the technical field relating to a flow cytometer or a cell sorter may be adopted.

The particles emitted from the chip may be sorted. For example, it is possible to generate a droplet containing one particle from an ejection port by vibrating the chip with a vibration element such as a piezo vibration element. It is possible to control a traveling direction and sort the particles by charging the droplet with a charging unit. Thus, the particle analysis system may be configured as a system having a sorting function.

Further, as the chip, a chip provided with a sorting mechanism in the chip may be used. An example of such a chip can include a microchip for sorting microparticles described in JP 2019-174192 A. With the chip, particles in a sample liquid can be sorted without contact with the outside air, that is, a closed type sorting operation becomes possible.

As described above, the particle analysis system may include a sorting unit for sorting particles. The sorting unit can sort particles on the basis of a fluorescence detection result of the detection unit.

The detection unit detects the light generated by the light irradiation unit irradiating the particles with light. For example, the detection unit may be configured to detect light generated by irradiating particles flowing in the flow path of the chip with light. The light detected by the detection unit is, for example, light containing fluorescence, and may be light containing fluorescence and light other than fluorescence. The detection unit 3 may be configured to further detect scattered light (for example, one or more of forward scattered light, back scattered light, and side scattered light), in addition to the detection of the fluorescence.

The detection unit includes at least one photodetector that detects light generated by the light irradiation unit irradiating particles with light. The number of photodetectors included in the detection unit may be, for example, 1 to 20, 1 to 15, or 1 to 10. Each photodetector includes one or more light reception elements and has, for example, a light reception element array. Each photodetector may include, for example, one or a plurality of photomultiplier tubes (PMTs) and/or photodiodes as the light reception elements, and in particular, includes one or a plurality of PMTs. The photodetector may include, for example, a PMT array in which a plurality of PMTs are arranged in a one-dimensional direction. The at least one photodetector may detect fluorescence and may be configured as a fluorescence detector.

The number of light reception elements (for example, the number of PMTs) included in each photodetector may be, for example, 2 or more, 5 or more, 8 or more, 10 or more, 15 or more, 20 or more, 22 or more, 24 or more, or 26 or more. The number of light reception elements (for example, the number of PMTs) included in each photodetector may be, for example, 50 or less, 45 or less, or 40 or less.

The detection unit may include a spectroscopic unit that disperses light. The spectroscopic unit may include, for example, a prism type spectroscopic optical element. The spectroscopic unit may be included in each photodetector. The spectroscopic unit, for example, may be configured to disperse light (for example, fluorescence) and cause light having a predetermined detection wavelength to reach a light reception element (for example, a PMT) to which the predetermined detection wavelength has been assigned.

The detection unit may include a signal processing unit. The signal processing unit converts an electrical signal obtained by the photodetector into a digital signal. The signal processing unit may include, for example, an A/D converter as a device that performs the conversion. An optical signal detected by the photodetector can be converted into a digital signal by the signal processing unit and transmitted to the information processing device, for example, via the input device. The digital signal is treated as optical data by the information processing device and can be a target of the unmixing processing. The optical data may include data regarding fluorescence intensity.

In the information processing system of the present technology configured as the particle analysis system, the processing unit of the information processing device processes optical data obtained by irradiating particles with light using the light irradiation unit. The processing may include the unmixing processing. The optical data may be optical data including, for example, fluorescence data. More specifically, the optical data may be light intensity data, and the light intensity may be light intensity data of light including fluorescence.

The processing unit preferably performs the unmixing processing using spectral reference data. Through this processing, it is possible to acquire the fluorescence intensity of each fluorescent dye from the optical data. Further, through this processing, leakage of the fluorescence that becomes a problem in a filter type flow cytometer of the related art is eliminated, and separation performance of the fluorescence is improved. According to the present technology, it is possible to prevent the deterioration in the performance of the unmixing processing by using an optimized combination of the antibody and the labeling fluorescent dye.

In the present specification, the spectral reference data (also referred to as SR data) is spectral data of fluorescence that is generated when each phosphor is irradiated with predetermined excitation light. The SR data can be obtained, for example, by the fluorescence detector detecting the fluorescence that has been generated by irradiating the particle labeled with each phosphor alone with predetermined excitation light.

The spectral reference data to be used in the unmixing processing includes spectral data of the fluorescence generated when the phosphor labeling the particle is irradiated with a predetermined excitation light. In order to obtain the spectral reference data to be used in the unmixing processing, for example, first, a particle population that is an analysis target is labeled with each of a plurality of phosphors labeling the particle population, and a plurality of single-stained particle populations are obtained. Next, spectral data of the fluorescence generated by light irradiation (particularly, laser light irradiation) is obtained for each of the plurality of single-stained particle populations. The obtained spectral data is used as the spectral reference data. Thus, the spectral data of the fluorescence regarding the particle population labeled by each of the plurality of phosphors can be used as the spectral reference data in the unmixing processing. This makes it possible to improve fluorescence separation performance.

The unmixing processing may be performed according to, for example, a fluorescence intensity correction method or a fluorescence intensity calculation method described in JP 2011-232259 A (PTL 1 above).

The unmixing processing can be performed by using, for example, a least square method (LSM), more preferably, a weighted least square method (WLSM). The unmixing processing using the least square method may be performed, for example, by using a fluorescence intensity correction method described in JP 5985140 B. The fluorescence intensity correction method can be performed by using, for example, Equation 94 of WLSM below.

[Math. 94]

$$
\begin{bmatrix} x_1 \\ \vdots \\ x_n \end{bmatrix} = ([S^T][L][S])^{-1}[S^T][L] \begin{bmatrix} y_1 \\ \vdots \\ y_m \end{bmatrix}
$$

$$
L = \begin{bmatrix} \lambda_1 & 0 & 0 \\ 0 & \ddots & 0 \\ 0 & 0 & \lambda_m \end{bmatrix}, \quad \lambda_i = \frac{1}{max(y_i,\ 0) + offset}
$$

In Equation 94 above, $x_n$ indicates fluorescence intensity of an n-th fluorescent dye, $[S^T]$ indicates a transpose matrix of the spectral reference, $[L]$ indicates a weight matrix, $[S]$ indicates a matrix of the spectral reference, $y_i$ indicates a measurement value in an i-th photodetector, $\lambda_i$ indicates a weight of an i-th photodetector, $max(y_i, 0)$ indicates a larger value when a detection value of the i-th detector is compared with zero, and offset' indicates a value determined on the basis of the detection value of each detector.

A fluorescence wavelength distribution of a phosphor (for example, a fluorescent dye) may be wide. Therefore, for example, a PMT to be used to detect fluorescence generated from a certain phosphor can also detect fluorescence generated from another phosphor. That is, the optical data acquired by each PMT may be data in which fluorescence data from a plurality of phosphors are superimposed. Therefore, correction for separating the optical data into fluorescence data from each phosphor is necessary. The unmixing processing is a scheme for the correction, data in which fluorescence data from a plurality of phosphors are superimposed is separated into fluorescence data from each phosphor by the unmixing processing, and fluorescence data from each phosphor can be obtained.

The processing unit generates output data using the fluorescence data obtained by the unmixing processing. The output data may be, for example, a two-dimensional plot regarding two desired phosphors among the plurality of phosphors used for labeling of the particle population, but the present invention is not limited thereto. A vertical axis of the two-dimensional plot may be fluorescence data (particularly, fluorescence intensity) of fluorescence corresponding to one of the two phosphors, and a horizontal axis may fluorescence data (particularly, fluorescence intensity) of fluorescence corresponding to the other phosphor. The two-dimensional plot may be, for example, a density plot (dot plot), a contour plot, or a plot of both a density and contour.

The particles may be, for example, particles having dimensions capable of flowing in a flow path provided in the chip. In the present technology, the particles may be appropriately selected by those skilled in the art. In the present technology, particles may include, for example, biological microparticles such as cells, a mass of cells, microorganisms, and liposomes, and synthetic microparticles such as gel particles, beads, latex particles, polymer particles, and industrial particles.

The biological microparticles (also referred to as biological particles) may include chromosomes, liposomes, mitochondria, organelles (cellular organelles), and the like that constitute various cells. Cells can include animal cells (such as blood cell lineage cells) and plant cells. The cells can be blood-based cells or tissue-based cells in particular. The blood-based cells may be floating-based cells such as T cells and B cells. The tissue-based cells may be, for example, adhesive cultured cells or adhesive cells separated from the tissue. The mass of cells can include, for example, spheroids and organoids. The microorganisms may include, for example, bacteria such as Escherichia coli, viruses such as tobacco mosaic virus, and fungi such as yeast. Further, the biological microparticles can also include biological macromolecules such as nucleic acids, proteins, and complexes thereof. These biological macromolecules, for example, may be extracted from cells or may be included in blood samples or other liquid samples. According to an embodiment of the present technology, the particles are biological particles, especially, cells.

The synthetic microparticles can be, for example, microparticles made of an organic or inorganic polymer material or a metal. The organic polymer materials may include polystyrene, styrene/divinylbenzene, polymethylmethacrylate, and the like. The inorganic polymer materials may include glass, silica, a magnetic material, and the like. The metal may include gold colloid gold, aluminum, and the like. The synthetic microparticles may be, for example, gel particles or beads, and may be, more particularly, gel particles or beads to which one or two or more combinations selected from oligonucleotides, peptides, proteins, and enzymes are bound.

The particle analysis system may set a particle population as an analysis target. In the particle analysis system, each particle included in the particle population may be irradiated with light by the light irradiation unit. The particle population may be, in particular, a population of biological particles, and more particularly, a population of cells.

The particle analysis system may set a particle population labeled with a plurality of phosphors as an analysis target. The phosphor that labels the particle population may be a fluorescent dye. Further, the fluorescent dye may be bound to particles (particularly, cells) via a molecule that specifically binds to the particles (for example, an antibody, an aptamer, DNA, or RNA, particularly an antibody).

The particle population may be included in the sample liquid when the light irradiation unit performs light irradiation. A type of the sample liquid may be appropriately selected by those skilled in the art, and may be determined depending on consideration factors such as a type of particles (cells).

The plurality of phosphors may be, for example, a plurality of dyes, particularly a plurality of fluorescent dyes. The phosphor may be, for example, a phosphor (dye) known in the technical field of flow cytometry. The particle population is, for example, a particle population labeled with five or more types of phosphors, and more preferably, may be a particle population labeled with eight or more, 10 or more, 15 or more, or 20 or more types of phosphors. The particle population may be a particle population labeled with 22 or more, 24 or more, or 26 or more types of phosphors. The particle population may be, for example, a particle population labeled with 50 or less, 45 or less, or 40 or less types of phosphors.

(2-3) Example of Processing in Information Processing System of Present Technology

An example of information processing in the information processing system 1 will be described hereinafter with reference to Figs. 5 to 8. Figs. 5 and 6 are as described in (2-1) above. Figs. 7 and 8 are examples of flow diagrams of the optimization processing of the fluorescent dye labeled antibody that is used in a particle analysis device such as a flow cytometer. In the optimization processing, the biomolecule may be an antigen or an antibody, and the phosphor may be a fluorescent dye.

In step S101 of Fig. 7, the processing unit 10 of the information processing device 2 starts the optimization processing.

In step S102, the information processing device 2 acquires the first data input by the user. More specifically, the processing unit 10 receives an input of the antigen list from the user. The antigen list may be an antigen desired to be set as a labeling target by the user with respect to a sample that is an analysis target of the particle analysis device. For example, the information processing device 2 causes the display device included in the output device 6 to display a screen for requesting selection of the antigen that is a labeling target of the particle analysis device. The user operates the input device 5 (for example, a mouse or a keyboard) to select an antigen that is the labeling target of the particle analysis device on the screen. The information processing device 2 receives the selected antigen as an antigen list.

In step S103, the information processing device 2 presents, to the user, a list of fluorescent marker candidates that can correspond to the respective antigens included in the antigen list input in step S102 (more specifically, a list of fluorescent dye labeling antibody candidates that specifically bind to the respective antigens). For example, the processing unit 10 causes the display device to display the list of the fluorescent marker candidates. The processing unit 10 may acquire information on the fluorescent marker from the database 3 in order to present the list of the fluorescent marker candidates, or may acquire the information on the fluorescent marker from the storage unit 13 inside the information processing device 2. The fluorescent marker list may be displayed on the basis of the information on the fluorescent marker.

In step S104, the information processing device 2 can acquire the identification information of the algorithm for acquiring the assignment information. The algorithm includes at least a combinatorial optimization algorithm that is executed by the combinatorial optimization processing unit.

More specifically, in step S104, the information processing device 2 presents a list of optimization processing candidates to the user. For example, the information processing device 2 presents, to the user, an optimization processing candidate list in which one or a plurality of other optimization processing, in addition to the optimization processing using the objective function described in (2-1) above, are listed as candidates. Accordingly, the information processing device 2 requests the user to select the type of optimization processing to be executed. In an example outside the scope of the invention as defined by the claims, the one or a plurality of other optimization processing may include, for example, optimization processing based on a rule or optimization processing based on total inspection. In the optimization processing based on a rule, good results are often obtained when the number of antigens included in the antigen list is small, but good results may not be obtained as the number increases. Further, the total inspection takes time because all combinations are examined. Thus, the optimization processing has advantages and disadvantages. Therefore, in the present step, the user is requested to select the optimization processing, making it possible to adopt appropriate processing according to a sample.

In step S105, the processing unit 10 executes the optimization processing selected in step S104. Hereinafter, a case in which the optimization processing using the objective function described in (2-1) above is selected in step S104 will be described. For description, Fig. 8 illustrating a more detailed flow of the optimization processing using the objective function will be referred to below.

In step S201 of Fig. 8, the processing unit 10 starts the optimization processing using the objective function.

In step S202, the processing unit 10 prepares the first data in which information on the antigen is listed, on the basis of the antigen list input in step S102. The antigen list itself may be treated as the first data.

In a preferred embodiment, in step S202, the first data may also include information (particularly, expression amount data) on an amount of each antigen included in the data. This makes an optimization processing in consideration of the amount of each antigen and the fluorescence intensity of the fluorescent dye possible.

In step S203, the processing unit 10 prepares the second data in which the information on the labeling phosphor is listed. In order to prepare the second data, the processing unit 10 can acquire information on the fluorescent marker bound to the antigen included in the first data prepared in step S202 from the database 3. When the storage unit 13 of the information processing device 2 has information on the fluorescent marker, the processing unit 10 may acquire the information from the storage unit 13 instead of the database 3.

The information on the fluorescent marker prepared in step S203 includes the fluorescence spectrum of each fluorescent marker, that is, fluorescence spectral data of the fluorescence dye constituting each fluorescent marker. Use of the fluorescence spectrum makes the optimization processing using the objective function possible.

The information on the fluorescent marker prepared in step S203 may include information on the antigen to which each fluorescent marker binds and/or information on a fluorescence level of each fluorescent marker. The former may be, for example, a name or abbreviation of the antigen or a number (ID number) of the antigen. The latter may include, for example, a luminance level, or may include a luminance value itself. The luminance is also referred to as fluorescence intensity. The luminance level may be, for example, information on which of a plurality of ranges the luminance belongs to, when a predetermined luminance range is divided into the plurality of ranges. The combination of the information on the fluorescence level with the information on the amount of each of the antigens makes it possible to use the objective function incorporating the dispersion of the fluorescence intensity described above, and to curb a variation in the fluorescence intensity of the selected fluorescent marker.

In step S204, the calculation processing unit 11 calculates an objective function regarding a combination of the biomolecule (particularly, an antigen or antibody) with the labeling phosphor on the basis of the first data prepared in step S202 and the second data prepared in step S203. The objective function is as described in "(2-1) Configuration Example of Information Processing System" above, and description thereof is also applied to the present calculation processing.

The calculation of the objective function may be performed, for example, by the calculation processing unit 11 executing a program for calculating the objective function, more particularly, a program for calculating the coefficient of the objective function.

The program may include, for example, a subroutine for calculating linear independence between the fluorescence spectra of the labeling phosphors assigned to respective biomolecules (particularly, antigens or antibodies) (hereinafter also referred to as a "linear independence subroutine"), and more specifically, may include a subroutine for calculating an objective function based on the determinant in Equation 8 described in "(1) Description of First Embodiment" above or a determinant equivalent thereto. More preferably, the subroutine may be a subroutine for calculating a term that enhances the linear independence between the fluorescence spectra of the labeling phosphors assigned to the respective biomolecules (particularly, antigens or antibodies).

An example of the linear independence subroutine is as described in Equation 95 below. This example is outside the scope of the invention as defined by the claims, and is retained for illustration purposes only.

[Math. 95]

```
def calc_linear_independence(spectrums, spins):
    objective = IsingObjective()
    for i in range(L): // L is number of candidates for fluorescent marker
      for j in range(L):
        rij = inner_product(spectrums[i], spectrums[j])
        objective += (1 - spins[i]) * (1 - spins[j]) * rij^2
      // In case of quadratic approximation of Taylor expansion of determinant
      //spin is assumed to take value of -1, 1
    return 0.125 * objective
```

The program may further include a subroutine regarding a constraint condition regarding the number of phosphors assigned to one antigen (hereinafter also referred to as a "phosphor number constraint subroutine"), in addition to the linear independence subroutine, and may include, preferably, a subroutine for calculating a constraint that one phosphor is assigned to one antigen.

An example of the phosphor number constraint subroutine is as described in Equation 96 below. This example is outside the scope of the invention as defined by the claims, and is retained for illustration purposes only.

[Math. 96]

```
def calc_matching_regularization(matching_conditions, spins):
    regularization = IsingObjective()
    k = 0
  for m in range(M):
    km = matching_conditions[m]
    // matching_conditions is the number km
of candidates for fluorescent marker for each
of M types of inspection target information
    sum = 0
    for i in range(km):
     sum += 1 - spins[i + k]
    regularization += (1 - 0.5 * sum)^2
    k += km
    return regularization
```

The program may further include a subroutine regarding a constraint condition regarding the dispersion of the fluorescence intensity (hereinafter also referred to as a "dispersion constraint subroutine"), in addition to the linear independence subroutine.

An example of the dispersion constraint subroutine is as described in Equation 97 below. This example is outside the scope of the invention as defined by the claims, and is retained for illustration purposes only.

[Math. 97]

```
def calc_amplitude_variance(matching_conditions, amplitudes, spins):
    regularization = IsingObjective()
    k = 0
    ams = []
    for m in range(M):
     km = matching_conditions[m]
     am = 0
     for i in range(km):
       am += amplitudes[i + k] * spins[i + k]
     ams.append(am)
    return calc_variance(ams) //Function of calculating variance
```

The program may include a subroutine for calculating the coefficient of the objective function (hereinafter also referred to as a "coefficient calculation subroutine") from an objective function obtained by executing the linear independence subroutine, an objective function obtained by executing two subroutines including the linear independence subroutine and the phosphor number constraint subroutine, or an objective function obtained by executing three subroutines including the linear independence subroutine, the phosphor number constraint subroutine, and the dispersion constraint subroutine.

More specifically, the coefficient calculation subroutine may be a subroutine for calculating the coefficient of the objective function of the binary variable in a quadratic format, particularly, an Ising coefficient.

An example of these objective functions is as described in "(1) Description of First Embodiment" above, and may be, for example, an objective function based on Equation 76 described in "(1) Description of First Embodiment" above. More preferably, the objective function may be an objective function obtained by converting Equation 76 into a binary quadratic format. The objective function converted into the binary quadratic format is, for example, the objective function of Equation 82 described in "(1) Description of First Embodiment" above. Further, the objective function may be an objective function based on Equation 76 to which an item regarding the dispersion of the fluorescence intensity has been added, may be, particularly, an objective function for which Equation 76 to which the item regarding the dispersion of the fluorescence intensity has been added is converted into a binary quadratic format, or may be, for example, the objective function of Equation 93 above.

An example of the coefficient calculation subroutine is as described in Equation 98 below. This example is outside the scope of the invention as defined by the claims, and is retained for illustration purposes only.

[Math. 98]

```
def calc ising_parameters(objective):
```

```
jij = matrix(L, L)
hk = matrix(L)
for i in range(L):
  for j in range(L):
    jij[i][j] = objective.get_jij(i, j)
for k in range(L):
  hk[k] = objective.get_hk(k)
return jij, hk
```

In some examples, the program includes the linear independence subroutine, the phosphor number constraint subroutine, the dispersion constraint subroutine, and the coefficient calculation subroutine. An example of a program including all four subroutines is as described in Equation 99 below. This example is outside the scope of the invention as defined by the claims, and is retained for illustration purposes only. Each subroutine included in Equation 99 below may be as shown in Equations 95 to 98 above.

[Math. 99]

```
def calc_linear_independence(spectrums, spins): ... return objective
def calc_matching_regularization(matching_matrix, spins): ... return matching_regularization
def calc_level_regularization(level_matrix, spins): ... return level_regularization
def calc_ising_parameters(objective): ... return jij, hk
def main():
  spectrums = load_spectrums(spectrum_file)
  matching_conditions = load_matching_conditions(matching_condition_file)
  level_conditions = load_level_conditions(level_condition_file)
  spins = IsingSpins(L)
  objective = calc_linear_independence(spectrums, spins)
  objective += lambda1 * calc_matching_regularization(matching_conditions, spins)
  objective += lambda2 * calc_level_regularization(level_conditions, spins)
  jij, hk = calc_ising_parameters(objective)
  spins = ising_solve(jij, hk)
  return 0.5 * (1 - spins)
```

In step S205, the information processing device 2 transmits the objective function calculated in step S204 to the calculation device 7 via the communication device 4. The information processing device 2 may transmit the coefficient of the objective function obtained by the calculation processing to the calculation device 7. The calculation device 7 may be an Ising machine specialized in optimization of the binary quadratic variable.

In step S206, the calculation device 7 receives the objective function.

In step S207, the combinatorial optimization processing unit included in the calculation device 7 generates combination information on the combination of the antigen (or antibody) with the labeling phosphor on the basis of the objective function, particularly, on the basis of the coefficient of the objective function. The combination information includes, for example, a binary variable selected on the basis of the coefficient of the objective function. More specifically, the calculation device 7 performs, for example, spin optimization using the coefficient of the objective function, thereby obtaining a combination of optimization spins. Thus, the combination information generated by the calculation device 7 includes spin variables, and is preferably an optimized combination of spin variables. The combination information may include one or more spin variable combinations.

In step S208, the calculation device 7 transmits the generated combination information to the information processing device 2.

In step S209, the information processing device 2 receives the combination information generated by the calculation device 7 via the communication device 4. For example, the information processing device 2 receives the value of the binary variable selected by the calculation device 7 that is an Ising machine.

In step S210, the assignment information output unit 12 generates information on assignment of the labeling phosphor to each antigen, on the basis of the combination information. For example, the assignment information output unit 12 acquires data regarding a combination of the biomolecule (particularly, an antigen or an antibody) and a labeling phosphor, on the basis of the spin variable acquired by the calculation device 7 that is an Ising machine. More specifically, the information processing device 2 acquires data regarding a combination of the biomolecule (particularly, an antigen or an antibody) with the labeling phosphor by converting the spin variable into a binary variable. The assignment information may include the data acquired in this way.

In step S211, the processing unit 10 ends the optimization processing.

In step S106, the assignment information output unit 12 outputs the assignment information generated in step S105 (particularly, step S210). For example, the assignment information output unit 12 may cause the output device 6 to output the assignment information, more specifically, may cause a display device that is the output device 6 to display the assignment information, or may cause a printing device that is the output device 6 to print the assignment information.

In step S107, the information processing device 2 receives a correction from the user regarding the output assignment information. That is, the user may correct a part of the combination of the output biomolecule (particularly, the antigen or antibody) and the labeling phosphor. Step S107 may be omitted.

In step S108, the processing unit 10 ends the optimization processing.

Through the above optimization processing, it is possible to automatically present an optimized combination of fluorescent markers to the user. Further, it is possible to prevent the performance of the unmixing processing from deteriorating through the combination of the fluorescent markers.

2. Second Embodiment (Information Processing Method)

The present technology also provides an information processing method. The information processing method includes a calculation processing step of calculating an objective function regarding a combination of a biomolecule, the biomolecule being a labeling target in a sample, and a labeling phosphor on the basis of first data listing information on the biomolecule and second data listing information on a fluorescence signal of the labeling phosphor; and an output step of outputting information on assignment of the labeling phosphors to respective biomolecules, the information being generated on the basis of combination information acquired from a combinatorial optimization processing unit on the basis of a coefficient of the objective function The information processing method may be performed for the assignment of the labeling phosphor to the biomolecule.

The information processing method according to the present technology may be performed, for example, as described in "(2-2) Example of Processing in Information Processing System" of "1. First Embodiment" above. Step S204 in the processing corresponds to the calculation processing step. Further, step S106 in the processing corresponds to the assignment information output step. The information processing method of the present technology may further include one or two or more of steps other than step S204 and step S106.

3. Third Embodiment (Program)

The present technology also provides a program for causing the information processing device to execute the information processing method (particularly, an optimization processing method) according to the present technology, and more particularly, a program for causing the information processing device to execute the calculation processing step and the output step described in "2. Second Embodiment (Information Processing Method)" above. The program may be recorded on, for example, any recording medium. The recording medium may be, for example, a microSD memory card, an SD memory card, a flash memory, a CD, a DVD, or a BD.

4. Fourth Embodiment (Information Processing Device)

The present technology also provides an information processing device including the calculation processing unit and the assignment information output unit described in "1. First Embodiment (Information Processing System)" above. By using the information processing device in combination with the combinatorial optimization processing unit, it is possible to automatically select a better combination of the biomolecule and the labeling phosphor.

5. Fifth Embodiment (Calculation Device)

As described in (1) of "1. First Embodiment (Information Processing System) above", the problem of optimum selection of the fluorescent marker of the flow cytometer can be formulated as the optimum selection problem of the basis vector candidate. In the present technology, combinatorial optimization processing is performed using the coefficient of the objective function formulated in this way. The combinatorial optimization processing can be executed by a calculation device such as an Ising machine, as described in "1. First Embodiment (Information Processing System)". That is, the present technology also provides a calculation device including a combinatorial optimization processing unit configured to execute combinatorial optimization processing using coefficients of an objective function formulated as the constrained optimum selection problem of a basis vector from basis vector candidates.

The calculation device including the combinatorial optimization processing unit can also be used in communication technology. The calculation device can be used, for example, to associate information or a transmission line with a waveform pattern. This will be described in more detail below in (1) to (3).

(1) Optimization of Communication Modulation Scheme

A communication path modulation scheme, particularly, a digital signal modulation scheme, is a scheme of assigning information (bits or bytes) to a waveform pattern of a communication carrier wave. Examples of currently well-known modulation schemes include amplitude modulation, frequency modulation, phase modulation, pulse modulation, or a combination of these modulation schemes.

For example, amplitude shift keying modulation (ASK) is a modulation scheme for changing an amplitude of a carrier wave for each time unit (clock) according to information in the time unit. In the modulation scheme, for example, when an information bit is 0, the amplitude is set to 0, and when the information bit is 1, the amplitude is fixed to 1. Phase shift keying (PSK) is a modulation scheme for changing a phase of a carrier wave according to information. In the modulation scheme, for example, when an information bit is 0, the phase is set to 0, and when the information bit is 1, the phase is fixed to 180 degrees. Quadrature phase amplitude shift keying (QAM) is a modulation scheme that is a combination of amplitude shift keying with phase shift keying. In the modulation scheme, a carrier wave having a predetermined phase and amplitude is assigned to the information.

Thus, associating the information with the waveform pattern is called a modulation scheme, particularly, a digital modulation scheme. In order to improve performance of the digital modulation scheme, it is preferable for a difference in the waveform patterns corresponding to different information to be large.

(2) Optimization of Communication Multiplexing Scheme

A Communication multiplexing, particularly, digital communication multiplexing scheme is a technology for simultaneously transporting a plurality of information sequences. Examples of well-known multiplexing schemes include time division multiplexing, frequency division multiplexing (frequency DM), and code division multiplexing (code DM).

The code division multiplexing is also called spectrum spreading, and includes frequency hopping and direct spreading (direct sequence). In the direct spreading, information (bit sequence) is further modulated with a pulse modulation pattern unique to the transmission line called a spreading code, and then the carrier wave modulation as usual (described above) is performed, so that a final modulation signal on the transmission line is generated. Here, interference of information in different transmission lines is prevented by changing the pulse modulation pattern of the spreading code depending on the transmission line. In order to improve performance of the multiplexing, it is preferable for waveform patterns corresponding to different spreading codes to differ, and for a combination of waveform patterns of a plurality of spreading codes not to match (a combination of) waveform patterns of other spreading codes.

(3) Combinatorial Optimization of Waveform Pattern

The combinatorial optimization processing in the present technology can be used for optimization of the communication modulation scheme or multiplexing scheme described in (1) and (2) above, and more specifically, can be used for association of the information or the transmission line to the waveform pattern.

Hereinafter, optimization of association between the information and the waveform pattern will be described.

For example, it is assumed that there are a plurality of patterns (for example, L types) that the waveform pattern can take, and some (for example, M types) of these are selected and assigned to the information. In this case, the M types of waveform patterns are indistinguishable when the M types of waveform patterns are the same, and are also indistinguishable even when combinations of the M types of waveform patterns become the same. Therefore, it is preferable for the M types of waveform patterns to be linearly independent. However, because the number of combinations for selecting M types of waveform patterns from L types of waveform patterns increases explosively as the number of L and M becomes enormous, it is expected that evaluation of the linear independence for each combination will take a long time.

According to the present technology, by implementing, on an Ising machine, an approximate equation of a determinant calculated from an L-dimensional square matrix that is generated from L types of waveform patterns, M-type waveform patterns satisfying linear independence are easily searched for. Hereinafter, the formulation for the implementation will be described.

Now, it is assumed that there are L waveform pattern candidates, that is, basis vector candidates, and the i-th basis vector is indicated by $u_i$. A value indicating whether or not to select the i-th basis vector $u_i$ is $b_i \in \{0, 1\}$ In this case, a determinant for evaluating the linear independence of the selected basis vector is as described in (1) of "1. First Embodiment (Information Processing System)" above, and is approximated as Equation 100 below.

[Math. 100]

$$L(\boldsymbol{b}) = -\left[1 - \frac{1}{2}\sum_{i=1}^{L}\sum_{k=1}^{L} b_i b_k r_{ik}^2 + \frac{1}{3}\sum_{i=1}^{L}\sum_{k=1}^{L}\sum_{l=1}^{L} b_i b_k b_l r_{ik} r_{il} r_{kl} + \cdots\right]$$

A function of Equation 99 is a sign-inverted version of the approximate equation (Equation 74) of the determinant, and preferably, has a smaller value. Constraints regarding how to select the basis vector are imposed on the binary value $b_i$. Because the number of selected basis vectors is M (M < L), constraints of Equation 101 below are imposed.

[Math. 101]

$$\sum_{i=1}^{L} b_i = M$$

By using Equation 100 and Equation 101 above, the optimum selection of the basis vector is formulated as Equation 102 below.

[Math. 102]

$$\boldsymbol{b}^* = \arg\min_{\boldsymbol{b}} \left[ L(\boldsymbol{b}) + \lambda \left( M - \sum_{i=1}^{L} b_i \right)^2 \right]$$

The conversion between the binary variable and the spin variable or a reduction of dimension of the spin variable in the objective function is as described in (1) of "1. First Embodiment (Information Processing System)" above.

As described above, it is possible to execute the optimization processing of association of information with the waveform pattern on the basis of the objective function of Equation 102, and more particularly, on the basis of the coefficient of the objective function obtained by converting the objective function of Equation 102 into a binary quadratic format.

Similarly, this can be used for association with a waveform pattern of the transmission line on the basis of the objective function of Equation 102, and more particularly, on the basis of the coefficient of the objective function obtained by converting the objective function of Equation 102 into the binary quadratic format.

That is, in an embodiment of the present technology, the combinatorial optimization processing unit included in the calculation device executes combinatorial optimization processing of a waveform pattern, on the basis of a coefficient of a combinatorial optimization objective function used to assign the waveform pattern to each piece of communication information on the basis of first data including communication information and second data including a waveform pattern of communication carrier waves. This makes it possible to, for example, increase the difference in the waveform patterns corresponding to the different information, and improve the performance of the digital modulation scheme.

In another embodiment of the present technology, the combinatorial optimization processing unit executes combinatorial optimization processing of a waveform pattern, on the basis of a coefficient of a combinatorial optimization objective function used to assign the waveform pattern to each spreading code on the basis of first data including a spreading code and second data including a waveform pattern of the spreading code. This makes it possible to make the waveform patterns corresponding to respective spreading codes different. Further, it is possible to prevent the combination of the waveform patterns of the plurality of spreading codes from matching the waveform patterns of the other spreading codes or a combination of the waveform patterns. Therefore, with the present technology, it is possible to improve performance of multiplexing.

[Reference Signs List]

| | |
|---|---|
| 1 | Information processing system |
| 2 | Information processing device |
| 3 | Database |
| 4 | Communication device |
| 5 | Input device |
| 6 | Output device |
| 7 | Calculation device |
| 10 | Processing unit |
| 11 | Calculation processing unit |
| 12 | Assignment information output unit |

**Claims**

1. An information processing device (2) included by an information processing system (1), the information processing device comprising:

   a calculation processing unit (11) configured to calculate an objective function regarding a combination of a biomolecule, the biomolecule being a labeling target in a sample, with a labeling phosphor, on the basis of first data listing information on the biomolecule, and second data listing information on the labeling phosphor; and an assignment information output unit (12) configured to output information on assignment of the labeling phosphors to respective biomolecules, the information being generated on the basis of combination information acquired from a combinatorial optimization processing unit on the basis of a coefficient of the objective function.

2. The information processing device according to claim 1, wherein the information processing device acquires the first data input by a user.

3. The information processing device according to claim 1, wherein the information processing device acquires the second data by referring to a database (3) on the basis of the first data, the database being configured to hold the second data.

4. The information processing device according to claim 1,

   wherein the information processing device acquires identification information of an algorithm for acquiring the assignment information, and the algorithm includes at least a combinatorial optimization algorithm executed by the combinatorial optimization processing unit.

5. The information processing device according to claim 1,

   wherein the objective function is an objective function formulated as a constrained optimum selection problem of basis vector candidates, and a candidate for the basis vector selected in the optimum selection problem corresponds to a labeling phosphor labeling a biomolecule, the biomolecule being a labeling target.

6. The information processing device according to claim 1, wherein the objective function is an objective function of a binary variable in a quadratic format.

7. The information processing device according to claim 6, wherein the objective function is configured to indicate linear independence between the fluorescence spectra of the labeling phosphors assigned to the respective biomolecules.

8. The information processing device according to claim 1, wherein the objective function is configured to include a constraint condition regarding the number of phosphors assigned to one biomolecule.

9. The information processing device according to claim 8, wherein the objective function is configured to include a constraint condition that one phosphor is assigned to one biomolecule.

10. The information processing device according to claim 1, wherein the objective function is configured to include a constraint condition regarding fluorescence intensity regarding a combination of a biomolecule and a phosphor assigned to the biomolecule.

11. The information processing device according to claim 10, wherein the fluorescence intensity is based on an amount of the biomolecule and the fluorescence intensity of the phosphor assigned to the biomolecule.

12. An information processing method comprising:

    a calculation processing step of calculating an objective function regarding a combination of a biomolecule, the biomolecule being a labeling target in a sample, and a labeling phosphor on the basis of first data listing information on the biomolecule and second data listing information on a fluorescence signal of the labeling phosphor; and

an output step of outputting information on assignment of the labeling phosphors to respective biomolecules, the information being generated on the basis of combination information acquired from a combinatorial optimization processing unit on the basis of a coefficient of the objective function.

13. A program for causing an information processing device (2) to execute:

a calculation processing step of calculating an objective function regarding a combination of a biomolecule, the biomolecule being a labeling target in a sample, and a labeling phosphor on the basis of first data listing information on the biomolecule and second data listing information on a fluorescence signal of the labeling phosphor; and
an output step of outputting information on assignment of the labeling phosphors to respective biomolecules, the information being generated on the basis of combination information acquired from a combinatorial optimization processing unit on the basis of a coefficient of the objective function.

14. An information processing system (1) comprising the information processing device (2) according to any of claims 1 to 11.

15. The information processing system according to Claim 14, further comprising: a database (3) being configured to hold the second data.

**Patentansprüche**

1. Informationsverarbeitungsvorrichtung (2), die in einem Informationsverarbeitungssystem (1) eingeschlossen ist, wobei die Informationsverarbeitungsvorrichtung umfasst:

eine Berechnungsverarbeitungseinheit (11), die dazu konfiguriert ist, eine Zielfunktion hinsichtlich einer Kombination eines Biomoleküls zu berechnen, wobei das Biomolekül ein Markierungsziel in einer Probe ist, mit einem Markierungsleuchtstoff auf der Basis erster Daten, die Informationen über das Biomolekül auflisten, und zweiter Daten, die Informationen über den Markierungsleuchtstoff auflisten; und
eine Zuweisungsinformationsausgabeeinheit (12), die dazu konfiguriert ist, Informationen zur Zuweisung der Markierungsleuchtstoffe zu jeweiligen Biomolekülen auszugeben, wobei die Informationen auf der Basis von Kombinationsinformationen erzeugt werden, die von einer Verarbeitungseinheit zur kombinatorischen Optimierung auf der Basis eines Koeffizienten der Zielfunktion erfasst werden.

2. Informationsverarbeitungsvorrichtung nach Anspruch 1, wobei die Informationsverarbeitungsvorrichtung die ersten von einem Benutzer eingegebenen Daten erfasst.

3. Informationsverarbeitungsvorrichtung nach Anspruch 1, wobei die Informationsverarbeitungsvorrichtung die zweiten Daten durch Bezugnahme auf eine Datenbank (3) auf der Basis der ersten Daten erfasst, wobei die Datenbank dazu konfiguriert ist, die zweiten Daten zu enthalten.

4. Informationsverarbeitungsvorrichtung nach Anspruch 1, wobei die Informationsverarbeitungsvorrichtung Identifikationsinformationen eines Algorithmus zum Erfassen der Zuweisungsinformationen erfasst und der Algorithmus mindestens einen Algorithmus zur kombinatorischen Optimierung einschließt, der von der Verarbeitungseinheit zur kombinatorischen Optimierung ausgeführt wird.

5. Informationsverarbeitungsvorrichtung nach Anspruch 1, wobei die Zielfunktion eine Zielfunktion ist, die als ein Optimumauswahlproblem von Basisvektorkandidaten unter Nebenbedingungen formuliert ist, und ein Kandidat für den im Optimumauswahlproblem ausgewählten Basisvektor einem Markierungsleuchtstoff entspricht, der ein Biomolekül markiert, wobei das Biomolekül ein Markierungsziel ist.

6. Informationsverarbeitungsvorrichtung nach Anspruch 1, wobei die Zielfunktion eine Zielfunktion einer binären Variablen in einem quadratischen Format ist.

7. Informationsverarbeitungsvorrichtung nach Anspruch 6, wobei die Zielfunktion dazu konfiguriert ist, eine lineare Unabhängigkeit zwischen den Fluoreszenzspektren der den jeweiligen Biomolekülen zugewiesenen Markierungsleuchtstoffen anzuzeigen.

**8.** Informationsverarbeitungsvorrichtung nach Anspruch 1, wobei die Zielfunktion dazu konfiguriert ist, eine Einschränkungsbedingung hinsichtlich der Anzahl der einem Biomolekül zugewiesenen Leuchtstoffe einzuschließen.

**9.** Informationsverarbeitungsvorrichtung nach Anspruch 8, wobei die Zielfunktion dazu konfiguriert ist, eine Einschränkungsbedingung einzuschließen, dass ein Leuchtstoff einem Biomolekül zugewiesen ist.

**10.** Informationsverarbeitungsvorrichtung nach Anspruch 1, wobei die Zielfunktion dazu konfiguriert ist, eine Einschränkungsbedingung hinsichtlich der Fluoreszenzintensität hinsichtlich einer Kombination aus einem Biomolekül und einem dem Biomolekül zugewiesenen Leuchtstoff einzuschließen.

**11.** Informationsverarbeitungsvorrichtung nach Anspruch 10,
wobei die Fluoreszenzintensität auf einer Menge des Biomoleküls und der Fluoreszenzintensität des dem Biomolekül zugewiesenen Leuchtstoffs basiert.

**12.** Informationsverarbeitungsverfahren, umfassend:

einen Berechnungsverarbeitungsschritt zum Berechnen einer Zielfunktion hinsichtlich einer Kombination eines Biomoleküls, wobei das Biomolekül ein Markierungsziel in einer Probe ist, und eines Markierungsleuchtstoffs auf der Basis erster Daten, die Informationen über das Biomolekül auflisten, und zweiter Daten, die Informationen zu einem Fluoreszenzsignal des Markierungsleuchtstoffs auflisten; und
einen Ausgabeschritt zum Ausgeben von Informationen zur Zuweisung der Markierungsleuchtstoffe zu jeweiligen Biomolekülen, wobei die Informationen auf der Basis von Kombinationsinformationen erzeugt werden, die von einer Verarbeitungseinheit zur kombinatorischen Optimierung auf der Basis eines Koeffizienten der Zielfunktion erfasst werden.

**13.** Programm, das eine Informationsverarbeitungsvorrichtung (2) veranlasst, Folgendes auszuführen:

einen Berechnungsverarbeitungsschritt zum Berechnen einer Zielfunktion hinsichtlich einer Kombination eines Biomoleküls, wobei das Biomolekül ein Markierungsziel in einer Probe ist, und eines Markierungsleuchtstoffs auf der Basis erster Daten, die Informationen über das Biomolekül auflisten, und zweiter Daten, die Informationen zu einem Fluoreszenzsignal des Markierungsleuchtstoffs auflisten; und
einen Ausgabeschritt zum Ausgeben von Informationen zur Zuweisung der Markierungsleuchtstoffe zu jeweiligen Biomolekülen, wobei die Informationen auf der Basis von Kombinationsinformationen erzeugt werden, die von einer Verarbeitungseinheit zur kombinatorischen Optimierung auf der Basis eines Koeffizienten der Zielfunktion erfasst werden.

**14.** Informationsverarbeitungssystem (1), umfassend die Informationsverarbeitungsvorrichtung (2) nach einem der Ansprüche 1 bis 11.

**15.** Informationsverarbeitungssystem nach Anspruch 14, ferner umfassend: eine Datenbank (3), die zum Enthalten der zweiten Daten konfiguriert ist.

**Revendications**

**1.** Dispositif de traitement d'informations (2) inclus dans un système de traitement d'informations (1), le dispositif de traitement d'informations comprenant :

une unité de traitement de calcul (11) configurée pour calculer une fonction objective concernant une combinaison d'une biomolécule, la biomolécule étant une cible de marquage dans un échantillon, avec un phosphore de marquage, sur la base de premières informations de listage de données sur la biomolécule et de secondes informations de listage de données sur le phosphore de marquage ; et
une unité de sortie d'informations d'attribution (12) configurée pour délivrer en sortie des informations sur l'attribution des phosphores de marquage à des biomolécules respectives, les informations étant générées sur la base d'informations de combinaison acquises à partir d'une unité de traitement d'optimisation combinatoire sur la base d'un coefficient de la fonction objective.

**2.** Dispositif de traitement d'informations selon la revendication 1, dans lequel le dispositif de traitement d'informations acquiert les premières données saisies par un utilisateur.

3. Dispositif de traitement d'informations selon la revendication 1, dans lequel le dispositif de traitement d'informations acquiert les secondes données en se référant à une base de données (3) sur la base des premières données, la base de données étant configurée pour contenir les secondes données.

4. Dispositif de traitement d'informations selon la revendication 1, dans lequel le dispositif de traitement d'informations acquiert des informations d'identification d'un algorithme pour l'acquisition des informations d'attribution, et l'algorithme comporte au moins un algorithme d'optimisation combinatoire exécuté par l'unité de traitement d'optimisation combinatoire.

5. Dispositif de traitement d'informations selon la revendication 1,
dans lequel la fonction objective est une fonction objective formulée comme un problème de sélection optimale sous contrainte de candidats vecteurs de base, et un candidat pour le vecteur de base sélectionné dans le problème de sélection optimale correspond à un phosphore de marquage marquant une biomolécule, la biomolécule étant une cible de marquage.

6. Dispositif de traitement d'informations selon la revendication 1, dans lequel la fonction objective est une fonction objective d'une variable binaire dans un format quadratique.

7. Dispositif de traitement d'informations selon la revendication 6, dans lequel la fonction objective est configurée pour indiquer une indépendance linéaire entre les spectres de fluorescence des phosphores de marquage attribués aux biomolécules respectives.

8. Dispositif de traitement d'informations selon la revendication 1, dans lequel la fonction objective est configurée pour comporter une condition de contrainte concernant le nombre de phosphores attribués à une biomolécule.

9. Dispositif de traitement d'informations selon la revendication 8, dans lequel la fonction objective est configurée pour comporter une condition de contrainte selon laquelle un phosphore est attribué à une biomolécule.

10. Dispositif de traitement d'informations selon la revendication 1, dans lequel la fonction objective est configurée pour comporter une condition de contrainte concernant une intensité de fluorescence d'une combinaison d'une biomolécule et d'un phosphore attribué à la biomolécule.

11. Dispositif de traitement d'informations selon la revendication 10, dans lequel l'intensité de fluorescence est basée sur une quantité de la biomolécule et sur l'intensité de fluorescence du phosphore attribué à la biomolécule.

12. Procédé de traitement d'informations comprenant :

une étape de traiment de calcul consistant à calculer une fonction objective concernant une combinaison d'une biomolécule, la biomolécule étant une cible de marquage dans un échantillon, et un phosphore de marquage sur la base de premières informations de listage de données sur la biomolécule et de secondes informations de listage de données sur un signal de fluorescence du phosphore de marquage ; et
une étape de sortie consistant à délivrer en sortie des informations sur l'attribution des phosphores de marquage aux biomolécules respectives, les informations étant générées sur la base d'informations de combinaison acquises à partir d'une unité de traitement d'optimisation combinatoire sur la base d'un coefficient de la fonction objective.

13. Programme permettant d'amener un dispositif de traitement d'informations (2) à exécuter :

une étape de traiment de calcul consistant à calculer une fonction objective concernant une combinaison d'une biomolécule, la biomolécule étant une cible de marquage dans un échantillon, et un phosphore de marquage sur la base de premières informations de listage de données sur la biomolécule et de secondes informations de listage de données sur un signal de fluorescence du phosphore de marquage ; et
une étape de sortie consistant à délivrer en sortie des informations sur l'attribution des phosphores de marquage aux biomolécules respectives, les informations étant générées sur la base d'informations de combinaison acquises à partir d'une unité de traitement d'optimisation combinatoire sur la base d'un coefficient de la fonction objective.

14. Système de traitement d'informations (1) comprenant le dispositif de traitement d'informations (2) selon l'une quelconque des revendications 1 à 11.

15. Système de traitement d'informations selon la revendication 14, comprenant en outre : une base de données (3) configurée pour contenir les secondes données.

Fig. 1

Fig. 2

Fig. 3

Fig. 4

Fig. 5

Fig. 6

Fig. 7

```
            ┌─────────────────────┐
            │        START        │ ～ S 1 0 1
            └─────────────────────┘
                      │
                      ▼
            ┌─────────────────────┐
            │ RECEIVE ANTIGEN LIST │ ～ S 1 0 2
            └─────────────────────┘
                      │
                      ▼
            ┌─────────────────────┐
            │ PRESENT FLUORESCENT  │ ～ S 1 0 3
            │     MARKER LIST      │
            └─────────────────────┘
                      │
                      ▼
            ┌─────────────────────┐
            │ PRESENT OPTIMIZATION │ ～ S 1 0 4
            │   PROCESSING LIST    │
            └─────────────────────┘
                      │
                      ▼
            ┌─┬─────────────────┬─┐
            │ │ EXECUTE OPTIMIZATION│ ～ S 1 0 5
            │ │    PROCESSING   │ │
            └─┴─────────────────┴─┘
                      │
                      ▼
            ┌─────────────────────┐
            │  OUTPUT ASSIGNMENT   │ ～ S 1 0 6
            │     INFORMATION      │
            └─────────────────────┘
                      │
                      ▼
            ┌─────────────────────┐
            │  RECEIVE CORRECTION  │ ～ S 1 0 7
            └─────────────────────┘
                      │
                      ▼
            ┌─────────────────────┐
            │         END         │ ～ S 1 0 8
            └─────────────────────┘
```

Fig. 8

## REFERENCES CITED IN THE DESCRIPTION

**Patent documents cited in the description**

- JP 2011232259 A
- WO 2019031048 A1
- US 2012056103 A1
- US 2016025621 A1
- US 2010012853 A1
- JP 2019174192 A
- JP 5985140 B

**Non-patent literature cited in the description**

- **CHIDOZIE C. ANYAEGBU**. *Optimisation of multiplex immunofluorescence for a non-spectral fluorescence scanning system*